(19) Europäisches Patentamt
European Patent Office
Office européen des brevets

(11) EP 3 349 856 B1

(12) **FASCICULE DE BREVET EUROPEEN**

(45) Date de publication et mention de la délivrance du brevet:
**22.02.2023 Bulletin 2023/08**

(21) Numéro de dépôt: **16766307.9**

(22) Date de dépôt: **16.09.2016**

(51) Classification Internationale des Brevets (IPC):
***A61Q 1/02*** *(2006.01)* ***A61Q 19/08*** *(2006.01)*
***A61K 8/11*** *(2006.01)* ***A61K 8/898*** *(2006.01)*
***A61K 8/06*** *(2006.01)* ***A61K 8/81*** *(2006.01)*
***B01J 13/10*** *(2006.01)* ***C09K 23/16*** *(2022.01)*
***C09K 23/54*** *(2022.01)*

(52) Classification Coopérative des Brevets (CPC):
**A61K 8/062; A61K 8/11; A61K 8/8147; A61K 8/898; A61Q 1/02; A61Q 19/08; B01J 13/10; C09K 23/16; C09K 23/54;** A61K 2800/5424; A61K 2800/5426; A61K 2800/594

(86) Numéro de dépôt international:
**PCT/EP2016/071924**

(87) Numéro de publication internationale:
**WO 2017/046299 (23.03.2017 Gazette 2017/12)**

(54) **EMULSIONS STABLES DE GOUTTES À ÉCORCE POLYMÉRIQUE**

STABILE EMULSIONEN VON POLYMERSCHALENTROPFEN

STABLE EMULSIONS OF POLYMER-SHELL DROPS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.09.2015 FR 1558847**

(43) Date de publication de la demande:
**25.07.2018 Bulletin 2018/30**

(73) Titulaire: **Capsum**
**13013 Marseille (FR)**

(72) Inventeurs:
- **GOUTAYER, Mathieu**
  **35400 Saint Malo (FR)**
- **PUJOL, Amélie**
  **13004 Marseille (FR)**

(74) Mandataire: **Lavoix**
**2, place d’Estienne d’Orves**
**75441 Paris Cedex 09 (FR)**

(56) Documents cités:
**WO-A1-2015/055839**
**WO-A1-2015/148892**
**FR-A1- 3 012 050**
**US-A1- 2014 045 949**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



EP 3 349 856 B1

## Description

La présente invention a pour objet des émulsions stables de type eau-dans-huile ou huile-dans-eau, notamment pour une application cosmétique. Elle a en outre pour objet des compositions, notamment cosmétiques, comprenant lesdites émulsions. Elle a également pour objet l'utilisation cosmétique desdites émulsions et/ou compositions, notamment pour le maquillage et/ou le soin des matières kératiniques, en particulier de la peau.

Dans le domaine cosmétique, il existe de nombreux types de compositions, notamment sous formes d'émulsions, en particulier stabilisées par des tensioactifs.

Cependant, la présence de tensioactifs ne permet pas d'envisager toutes les formulations possibles. Par ailleurs, de par leurs effets potentiellement irritants, les utilisateurs sont de plus en plus à la recherche de produits cosmétiques dénués de tels tensioactifs. De plus, les utilisateurs de compositions cosmétiques recherchent de plus en plus des compositions faciles à appliquer sur la peau et présentant des propriétés cosmétiques satisfaisantes, notamment en termes de texture. Enfin, la mise à disposition d'émulsions obtenues au moyen de procédés de préparation simples demeure un objectif constant.

WO/2012/120043 décrit un procédé de formation de gouttes stabilisées par coacervation complexe biphasique, notamment par microfluidique. Ce procédé simple conduit à la formation d'une pluralité de gouttes stables stabilisées par une écorce très fine.

US 2014/0045949 décrit un procédé de formation de gouttes d'une première phase dispersée dans une deuxième phase sensiblement immiscible avec encapsulation de la phase dispersée par formation d'un coacervat à partir de deux polymères précurseurs, l'un des deux polymères étant un polymère lipophile cationique contenant une silicone et une fonction amine primaire, secondaire ou tertiaire comme l'amodiméthicone et ses dérivés, le second étant un polymère hydrophile anionique tel qu'un copolymère d'acide acrylique ou d'acide maléique ou encore un copolymère alkyl/$C_{10}$-$C_{30}$ alkyl acrylates.

Néanmoins, le développement d'émulsions présentant des caractéristiques améliorées, notamment en termes de résistance mécanique et de coalescence, demeure un objectif constant.

La présente invention a ainsi pour but de fournir une émulsion cinétiquement stable, par exemple à température ambiante ou à température élevée, notamment jusqu'à 50°C.

Par « cinétiquement stable », au sens de la présente invention, on entend désigner une émulsion stable dans le temps, c'est-à-dire pour laquelle une homogénéité visuelle de la phase dispersée dans la phase continue demeure satisfaisante ou, en d'autres termes, pour laquelle on n'observe pas de déphasage.

La présente invention a également pour but de fournir une émulsion présentant des propriétés améliorées en termes de résistances mécaniques, lui permettant notamment de résister à des cisaillements ou fragmentations des gouttes, par exemple lors de l'industrialisation ou du transport de ladite émulsion ou de compositions ou produits cosmétiques la contenant.

La présente invention a encore pour but de fournir une émulsion présentant des propriétés améliorées en termes de résistance à la coalescence.

La présente invention a en outre pour but de fournir une émulsion cosmétique présentant des propriétés cosmétiques satisfaisantes.

Ainsi, la présente invention concerne une émulsion stable, de type eau-dans-huile ou huile-dans-eau, comprenant une phase continue et une phase dispersée sous forme de gouttes, lesdites gouttes comprenant une écorce formée d'au moins un polymère anionique comprenant au moins une fonction acide carboxylique et d'au moins un polymère cationique comprenant au moins deux fonctions amines, dans laquelle la quantité de fonctions amines apportées par le polymère cationique, dans la phase considérée, est comprise entre 0,2 μmol et 2 μmol par gramme de ladite phase,

le polymère cationique étant un polymère silicone modifié par une fonction amine primaire, secondaire ou tertiaire, et la phase grasse ne comprenant pas de polydiméthylsiloxane.

Selon un mode de réalisation, la présente invention concerne une émulsion de type huile-dans-eau, comprenant une phase continue aqueuse et une phase grasse dispersée sous forme de gouttes, lesdites gouttes comprenant une écorce formée d'au moins un polymère anionique comprenant au moins une fonction acide carboxylique et d'au moins un polymère cationique comprenant au moins deux fonctions amines, dans laquelle la quantité de fonctions amines apportées par le polymère cationique, dans la phase grasse, est comprise entre 0,2 μmol et 2 μmol par gramme de phase grasse,

le polymère cationique étant un polymère silicone modifié par une fonction amine primaire, secondaire ou tertiaire, et la phase grasse ne comprenant pas de polydiméthylsiloxane.

Selon un autre mode de réalisation, la présente invention concerne une émulsion de type eau-dans-huile,

comprenant une phase grasse continue et une phase dispersée aqueuse sous forme de gouttes, lesdites gouttes comprenant une écorce formée d'au moins un polymère anionique comprenant au moins une fonction acide carboxylique et d'au moins un polymère cationique comprenant au moins deux fonctions amines, dans laquelle la quantité de fonctions amines apportées par le polymère cationique, dans la phase grasse, est comprise entre 0,2 μmol et 2 μmol par gramme de phase grasse,

le polymère cationique étant un polymère silicone modifié par une fonction amine primaire, secondaire ou tertiaire, et la phase grasse ne comprenant pas de polydiméthylsiloxane.

La présente demande décrit également des compositions dans lesquelles la quantité de fonctions amines apportées par le polymère cationique, dans la phase grasse, est comprise entre 0,3 μmol et 7 μmol, de préférence entre 0,4 μmol et 5 μmol, en particulier entre 0,8 μmol et 3 μmol, et mieux entre 0,8 μmol et 2 μmol, par gramme de phase grasse.

Dans le cadre de l'invention, et sauf mention contraire, on entend par « fonction amine », une fonction $-NH_2$.

En effet, dans le contexte d'une émulsion stabilisée par un coacervat résultant de l'interaction entre un polymère anionique, notamment un carbomère, et un polymère cationique, notamment une amodiméthicone, les inventeurs ont observé que la densité en fonctions amines apportées par le polymère cationique au niveau de la phase grasse impacte les propriétés des gouttes formant ladite émulsion en termes de résistance mécanique, notamment de résistance aux cisaillements ou à la fragmentation, et de résistance à la coalescence.

Dans le cadre de l'invention, et sauf mention contraire, on entend par « quantité de fonctions amines dans la phase grasse », la quantité totale de fonctions (ou groupes ou groupements) amines présentes dans la phase grasse. Il peut par exemple s'agir de fonctions amines apportées par le polymère cationique ou par tout autre composé présent dans la phase grasse. En effet, la phase grasse peut comprendre, outre le polymère cationique, des composés différents du polymère cationique et qui comprennent au moins une fonction amine, tel que par exemple le N-acétylglucosamine.

Dans le cadre de l'invention, et sauf mention contraire, on entend par « quantité de fonctions amines apportées par le polymère cationique, dans la phase grasse», la quantité de fonctions (ou groupes ou groupements) amines présentes dans la phase grasse qui sont portées par (ou présentes sur) le polymère cationique uniquement. Ainsi, la quantité de fonctions amines apportées par le polymère cationique, dans la phase grasse, ne comprend pas la quantité de fonctions amines portées par tout autre composé différent du polymère cationique dans cette phase grasse.

Pour des raisons évidentes, les fonctions amines apportées par le polymère cationique considérées sont celles aptes à réagir avec le polymère anionique, en particulier avec les groupes carboxyliques portés par ledit polymère anionique.

Ainsi, avantageusement, au moins 50%, de préférence au moins 60%, en particulier au moins 70%, mieux au moins 80%, de préférence au moins 90%, et tout particulièrement au moins 99%, des fonctions amines apportées par (ou présentes sur) le polymère cationique sont aptes à réagir avec le polymère anionique, en particulier avec les groupes carboxyliques portés par ledit polymère anionique.

Les dispersions selon l'invention sont avantageusement stables, à température ambiante ou élevée (par exemple jusqu'à 50°C). Elles présentent en outre une coalescence faible, voire nulle. Ainsi, les gouttes de la dispersion peuvent conserver leur aspect esthétique attrayant recherché par le consommateur. Cette propriété intéressante en termes de stabilité cinétique est d'autant plus inattendue que l'écorce des gouttes, décrite en détail ci-après, est très fine. Ainsi, au moment de l'application sur une matière kératinique, aucune résistance attachée à la rupture de l'écorce n'est ressentie par l'utilisateur et aucun dépôt résiduel de ladite écorce n'est par ailleurs constaté. On parle ainsi d'écorce évanescente.

Les gouttes d'une dispersion selon l'invention, par la nature et les propriétés de leurs écorces, diffèrent donc de capsules solides, c'est-à-dire des capsules dotées d'une membrane solide, telle que par exemple celles décrites dans WO 2010/063937.

En outre, les dispersions selon l'invention présentent avantageusement des résistances mécaniques élevées, ce qui permet d'éviter des cisaillements ou fragmentations des gouttes, notamment lors de leur industrialisation ou encore leur transport.

Dans le cadre de la présente invention, les émulsions susmentionnées peuvent être désignées indifféremment par le terme "dispersions".

Selon l'invention, le pH d'une émulsion est typiquement compris entre 5,5 et 7,0.

Une goutte selon l'invention est composée d'un coeur, aussi appelé intérieur de la goutte, entouré d'une écorce, qui isole l'intérieur de la goutte de la phase continue de l'émulsion.

Selon un mode de réalisation, les émulsions selon l'invention ne comprennent pas de tensioactif. Elles se différencient donc, dans ce cas, des émulsions/compositions cosmétiques usuelles.

Selon un mode de réalisation, une émulsion selon l'invention est préparée par mise en oeuvre d'un procédé « non-microfluidique », à savoir par simple émulsification. Selon ce mode de réalisation, la taille des gouttes de la phase

dispersée est inférieure à 500 μm, voire inférieure à 200 μm. Préférentiellement, la taille des gouttes est comprise entre 0,5 μm à 50 μm, de préférence entre 1 μm et 20 μm.

Selon ce mode de réalisation, la présente invention permet de disposer de gouttes de taille réduite, notamment par rapport à des gouttes obtenues par un procédé microfluidique. Cette petite taille de gouttes va avoir un effet sur la texture. En effet, une émulsion selon l'invention, formée de gouttes finement dispersées, présente des qualités d'onctuosité améliorée.

Selon un autre mode de réalisation, une émulsion selon l'invention est préparée par mise en oeuvre d'un procédé « microfluidique », notamment tel que décrit ci-après. Selon ce mode de réalisation, la taille des gouttes de la phase dispersée est supérieure à 500 μm, voire supérieure à 1000 μm. Préférentiellement, selon ce mode de réalisation, la taille des gouttes est comprise entre 500 et 3 000 μm, de préférence entre 1000 μm et 2000 μm. A ce titre, il n'était pas évident que les compositions comprenant de telles gouttes de taille supérieure à 500 μm soient stables.

Dans le cadre de la présente invention, le terme "taille" désigne le diamètre, notamment le diamètre moyen, des gouttes.

Selon un mode de réalisation, une émulsion selon l'invention est de type huile-dans-eau.

Selon un autre mode de réalisation, une émulsion selon l'invention est de type eau-dans-huile.

***Viscosité***

La viscosité des émulsions selon l'invention peut varier de façon importante ce qui permet donc d'obtenir des textures variées.

Selon un mode de réalisation, l'émulsion selon l'invention a une viscosité comprise de 1 mPa.s à 500 000 mPa.s, de préférence de 10 à 300 000 mPa.s, et encore plus préférentiellement de 1 000 mPa.s à 100 000 mPa.s, telle que mesurée à 25°C.

La viscosité est mesurée à température ambiante, par exemple T=25°C ± 2°C et à pression ambiante, par exemple 1013 mbar, par la méthode décrite ci-après.

On utilise un viscosimètre de type Brookfield, typiquement un viscosimètre numérique Brookfield RVDV-E (couple de torsion du ressort de 7187,0 dyne-cm), qui est un viscosimètre rotationnel à vitesse imposée muni d'un mobile (désigné par le terme anglais « Spindle »). Une vitesse est imposée au mobile en rotation et la mesure du couple exercé sur le mobile permet de déterminer la viscosité en connaissant les paramètres de géométrie/forme du mobile utilisé.

On utilise par exemple un mobile de taille No. 04 (référence Brookfield: RV4). Le taux de cisaillement correspondant à la mesure de la viscosité est défini par le mobile utilisé et la vitesse de rotation de celui-ci.

La mesure de viscosité est effectuée sur 1 minute à température ambiante (T=25°C ± 2°C). On place environ 150 g de solution dans un bécher de 250 ml de volume, ayant un diamètre d'environ 7 cm de façon à ce que la hauteur du volume occupée par les 150 g de solution soit suffisante pour arriver à la jauge marquée sur le mobile. Ensuite, on démarre le viscosimètre sur une vitesse de 10 tours/min et on attend que la valeur affichée sur l'écran soit stable. Cette mesure donne la viscosité du fluide testé, telle que mentionnée dans le cadre de la présente invention.

***Phase grasse***

Selon un mode de réalisation, les émulsions selon l'invention comprennent une phase grasse, notamment dispersée, sous forme de gouttes.

La phase grasse d'une émulsion selon l'invention peut comprendre au moins une huile et/ou au moins un corps gras solides à température et pression ambiante, notamment tels que définis ci-après.

*Huile(s)*

Selon un mode de réalisation, les gouttes de la phase dispersée peuvent comprendre au moins une huile. La phase grasse dispersée peut donc être désignée comme une phase huileuse.

Selon un mode de réalisation, la phase grasse comprend une huile H1 dans laquelle le polymère cationique est soluble. En particulier, l'émulsion selon l'invention comprend au moins une huile compatible avec le polymère cationique. L'huile H1 correspond par exemple à un bon solvant du polymère cationique.

Les émulsions selon l'invention peuvent comprendre une seule huile H1 ou un mélange de plusieurs huiles H1. Une émulsion selon l'invention peut donc comprendre au moins une, au moins deux, au moins trois, au moins quatre, au moins cinq, voire plus, d'huile(s) H1 telle(s) que décrite(s) ci-après.

On entend par « huile » un corps gras liquide à la température ambiante (25°C).

Comme huiles H1 utilisables dans l'émulsion de l'invention, on peut citer par exemple :

- les huiles hydrocarbonées d'origine animale, telles que le perhydrosqualène et le squalane ;

- les esters et les éthers de synthèse, notamment d'acides gras, comme les huiles de formules $R_1COOR_2$ et $R_1OR_2$ dans laquelle $R_1$ représente le reste d'un acide gras en $C_8$ à $C_{29}$, et $R_2$ représente une chaîne hydrocarbonée, ramifiée ou non, en $C_3$ à $C_{30}$, comme par exemple l'huile de Purcellin, l'isononanoate d'isononyle, le néopentanoate d'isodécyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétrabéhénate de pentaérythrityle (DUB PTB) ou le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les huiles de silicone, comme par exemple les polyméthylsiloxanes (PDMS) volatiles ou non à chaine siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaine siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les alcools gras ayant de 8 à 26 atomes de carbone, comme l'alcool cétylique, l'alcool stéarylique et leur mélange (alcool cétylstéarylique), ou encore l'octyldodécanol ;
- les huiles fluorées partiellement hydrocarbonées et/ou siliconées comme celles décrites dans le document JP-A-2-295912 ; et
- leurs mélanges.

Selon un mode de réalisation, l'huile H1 est choisie parmi les esters de formule $R_1COOR_2$, dans laquelle $R_1$ représente le reste d'un acide gras en $C_8$ à $C_{29}$, et $R_2$ représente une chaîne hydrocarbonée, ramifiée ou non, en $C_3$ à $C_{30}$.

Selon un mode de réalisation, l'huile H1 est choisie parmi les alcools gras ayant de 8 à 26 atomes de carbone.

Selon un mode de réalisation, l'huile H1 est choisie parmi les huiles hydrocarbonées ayant de 8 à 16 atomes de carbone, et notamment les alcanes ramifiés en $C_8$-$C_{16}$ (appelées aussi isoparaffines ou isoalcanes), comme l'isododécane (encore appelé 2,2,4,4,6-pentaméthylheptane), l'isodécane, l'isohexadécane, et, par exemple, les huiles vendues sous les noms commerciaux d'Isopars®ou de Permethyls®.

Selon un mode de réalisation préféré, l'huile H1 est choisie dans le groupe constitué de l'isononanoate d'isononyle, de la diméthicone, de l'isohexadécane, du polydiméthylsiloxane, de l'octyldodécanol, du néopentanoate d'isodécyle et de leurs mélanges.

De préférence, l'huile H1 est l'isononanoate d'isononyle.

Selon un mode de réalisation, l'huile H1 n'est pas une huile végétale.

Selon un mode de réalisation, l'huile H1 n'est pas figurée par le polydiméthylsiloxane (PDMS), et de préférence n'est pas une huile de silicone.

Selon l'invention, la phase grasse des gouttes ne comprend pas de polydiméthylsiloxane (PDMS), et de préférence ne comprend pas d'huile de silicone.

Selon un mode de réalisation préféré, une émulsion selon l'invention comprend au moins 1% en poids d'huile(s) H1, de préférence d'isononanoate d'isononyle, par rapport au poids total de ladite émulsion.

Selon un mode de réalisation, la teneur en huile(s) H1 dans la phase grasse est comprise entre 1% et 99,99%, de préférence entre 20% et 90%, et en particulier entre 50% et 80%, en poids par rapport au poids total de ladite phase grasse.

Selon un mode de réalisation, la phase grasse des émulsions de l'invention peut comprendre en outre au moins une huile hydrocarbonée d'origine végétale H2. La phase grasse peut comprendre plusieurs huiles H2.

Comme huiles végétales H2, on peut notamment citer les triglycérides liquides d'acides gras en $C_4$-$C_{10}$ comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux commercialisés par la société Stearineries Dubois ou ceux disponibles sous les dénominations commerciales « Miglyol 810 », « Miglyol 812 » et « Miglyol 818 » par la société Dynamit Nobel, l'huile de jojoba, ou l'huile de beurre de karité.

Parmi les huiles H2, on peut également mentionner les composés suivants : les triglycérides en C10-C18 liquides à la température ambiante (25°C), les triglycérides d'acides caprylique et caprique (nom INCI : Caprylic/Capric Triglycéride), les triglycérides d'acides caprylique, caprique, myristique et stéarique (nom INCI : Caprylic/capric/myristic/stearic Triglyceride), le triéthylhexanoine, l'huile végétale hydrogénée, l'huile de graine de limnanthe *Limnanthes Alba* (nom INCI : Limnanthes Alba (Meadowfoam) Seed Oil), l'huile d'olive *Olea Europaea* (nom INCI : Olea Europaea

(Olive) Fruit Oil), l'huile de noix de macadamia (nom INCI : Macadamia Ternifolia Seed Oil), l'huile d'églantier *Rosa Canina* (nom INCI : Rosa Canina Fruit Oil), l'huile de soja (nom INCI : Glycine Soja (Soybean) Oil), l'huile de graines de tournesol (nom INCI : Helianthus Annuus (Sunflower) Seed Oil), l'huile de maïs (nom INCI : Zea Mays (Corn) Oil), l'huile de palme hydrogénée (nom INCI : Hydrogenated Palm Oil), le tribéhénine (nom INCI : tribehenin), le triisostéarine (nom INCI : triisostearin), l'huile de noyau d'abricot (nom INCI : Prunus Armeniaca (Apricot) Kernel Oil), l'huile de son de riz (nom INCI : Oryza Sativa (Rice) Bran Oil), l'huile d'argan (nom INCI : Argania Spinosa Kernel Oil), l'huile d'avocat (nom INCI : Persea Gratissima Oil), l'huile d'onagre (nom INCI : Oenothera Biennis Oil), l'huile de palme (nom INCI : Elaeis Guineensis Oil), l'huile de germe de riz (nom INCI : Oryza Sativa Germ Oil), l'huile de noix de coco hydrogénée (nom INCI : Hydrogenated Coconut Oil), l'huile d'amande douce (nom INCI : Prunus Amygdalus Dulcis Oil), l'huile de pépins de raisin (nom INCI : Vitis Vinifera Seed Oil), l'huile de graine de sésame (nom INCI : Sesamum Indicum Seed Oil), l'huile de graine d'arachide (nom INCI : Arachis Hypogaea Oil), l'huile de colza hydrogénée (nom INCI : Hydrogenated Rapeseed Oil), l'huile de *Mortierella isabellina* (nom INCI : Mortierella Oil), l'huile de graine de carthame (nom INCI : Carthamus Tinctorius Seed Oil), l'huile de noix du Queensland *Macadamia integrifolia* (nom INCI : Macadamia Integrifolia Seed Oil), le tricaprylin (ou triacylglycérol), l'huile végétale (nom INCI : Olus Oil), l'huile de palme extraite du noyau (nom INCI : Elaeis Guineensis Kernel Oil), l'huile de noix de coco (nom INCI : Cocos Nucifera Oil), l'huile de germe de blé (nom INCI : Triticum Vulgare Germ Oil), l'huile de graine de bourrache (nom INCI : Borago Officinalis Seed Oil), l'huile de karité (nom INCI : Butyrospermum Parkii Oil), l'huile de noisette (nom INCI : Corylus Avellana Seed Oil), l'huile de ricin hydrogénée (nom INCI : Hydrogenated Castor Oil), l'huile de noyau de palme hydrogénée (nom INCI : Hydrogenated Palm Kernel Oil), l'huile de graine de mangue (nom INCI : Mangifera Indica Seed Oil), l'huile de graine de grenadier (nom INCI : Punica Granatum Seed Oil), l'huile de graine de chou chinois (nom INCI : Brassica Campestris Seed Oil), l'huile de graine de fruit de la passion (nom INCI : Passiflora Edulis Seed Oil), l'huile de graine de camélia du Japon (nom INCI : Camellia Japonica Seed Oil), l'huile de graine de thé vert (nom INCI : Camellia Sinensis Seed Oil), l'huile de germe de maïs (nom INCI : Zea Mays Germ Oil), l'huile d'hoplostèthe (nom INCI : Hoplostethus Oil), l'huile de noix du Brésil (nom INCI : Bertholletia Excelsa Seed Oil), l'huile de graine de rosier musqué (nom INCI : Rosa Moschata Seed Oil), l'huile de graine d'Inca Inchi (ou Sacha Inchi)(nom INCI : Plukenetia Volubilis Seed Oil), l'huile de graine de Babassu (nom INCI : Orbignya Oleifera Seed Oil), l'huile de graines d'une souche hybride de tournesol (nom INCI : Helianthus Annuus Hybrid Oil), l'huile d'argousier (nom INCI : Hippophae Rhamnoides Oil), l'huile de graine de *Marula* (nom INCI : Sclerocarya Birrea Seed Oil), l'huile de graine de *Aleurites Moluccana* (nom INCI : Aleurites Moluccana Seed Oil), l'huile de graine de rosier rubigineux (nom INCI : Rosa Rubiginosa Seed Oil), l'huile de graine de *Camellia Kissi* (nom INCI : Camellia Kissi Seed Oil), l'huile de graine de baobab (nom INCI : Adansonia Digitata Seed Oil), l'huile de baobab (nom INCI : Adansonia Digitata Oil), l'huile de graine de *Moringa* (nom INCI : Moringa Pterygosperma Seed Oil), l'huile de gaine de périlla (nom INCI : Perilla Ocymoides Seed Oil), l'huile de graine de ricin (nom INCI : Ricinus Communis Seed Oil), l'huile de canola (nom INCI : Canola Oil), l'huile de graine de cassis (nom INCI : Ribes Nigrum Seed Oil), l'huile de graine de thé (nom INCI : Camellia Oleifera Seed Oil), l'huile de graine de framboise (nom INCI : Rubus Idaeus Seed Oil), l'huile de graine de crambe d'Abyssinie (nom INCI : Crambe Abyssinica Seed Oil), l'huile de graine d'églantier (nom INCI : Rosa Canina Seed Oil), l'huile de vipérine à feuilles de plantain (nom INCI : Echium Plantagineum Seed Oil), l'huile de graine de tomate (nom INCI : Solanum Lycopersicum Seed Oil), l'huile essentielle d'amande amère (nom INCI : Prunus Amygdalus Amara Kernel Oil), l'huile de graine de yuzu (nom INCI : Citrus Junos Seed Oil), l'huile de graine de courge (nom INCI : Cucurbita Pepo Seed Oil), l'huile de vison *Mustela Mustelidae* (nom INCI : Mustela Oil), l'huile de graine de dattier du désert (nom INCI : Balanites Roxburghii Seed Oil), l'huile de graine de *Brassica Napus* (nom INCI : Brassica Napus Seed Oil), l'huile de calophylle (nom INCI : Calophyllum Inophyllum Seed Oil), l'huile de graine de mure arctique (nom INCI : Rubus Chamaemorus Seed Oil), l'huile de graine de pin blanc du Japon (nom INCI : Pinus Pentaphylla Seed Oil), l'huile de graine de pastèque (nom INCI : Citrullus Lanatus Seed Oil), l'huile de graine de noix (nom INCI : Juglans Regia Seed Oil), l'huile de graine de nigelle (nom INCI : Nigella Sativa Seed Oil), l'huile de graine de carotte (nom INCI : Daucus Carota Sativa Seed Oil), l'huile de graine de *Coix Lacryma-jobi Ma-yuen* (nom INCI : Coix Lacryma-jobi Ma-yuen Seed Oil), l'huile de de graine de *Coix Lacryma-jobi* (nom INCI : Coix Lachryma-jobi Seed Oil), le mélange de lipides de la farine de *Triticum Vulgare* (nom INCI : Triticum Vulgare Flour Lipids), le trihydroxyméthoxystéarine (nom INCI : Trihydroxymethoxystearin), le triheptanoine (nom INCI : Triheptanoin), l'huile de graine de canneberge (nom INCI : Vaccinium Macrocarpon Seed Oil), l'huile de vanille (nom INCI : Vanilla Planifolia Fruit Oil), l'huile de graine de canneberge (nom INCI : Oxycoccus Palustris Seed Oil), l'huile d'Açaï (nom INCI : Euterpe Oleracea Fruit Oil), le triester d'huile de ricin hydrogénée et de l'acice isostéarique (nom INCI : Hydrogenated Castor Oil Triisostearate), l'huile de coton hydrogénée (nom INCI : Hydrogenated Cottonseed Oil), l'huile d'olive hygrogénée (nom INCI : Hydrogenated Olive Oil), l'huile d'arachide hydrogénée (nom INCI : Hydrogenated Peanut Oil), l'huile de soja hydrogénée (nom INCI : Hydrogenated Soybean Oil), l'huile extraite de jaune d'oeuf de poule (nom INCI : Egg Yolk Oil), l'huile de noyau de noyau de pêche (nom INCI : Prunus Persica Kernel Oil), les glycérides d'huile de canola et de phytostérols (nom INCI : Phytosteryl Canola Glycerides), l'huile de graine de cassissier (nom INCI : Ribes Nigrum (Black Currant) Seed Oil), l'huile de graine de karanja (nom INCI : Pongamia Glabra Seed Oil) et l'huile de roucou (nom INCI : Roucou (Bixa orellana) Oil), l'extrait d'huile d'olive, notamment le phytosqualane, l'huile de rosier muscat, l'huile

de coriandre, l'huile de lin, l'huile de chia, l'huile de fénugrec, l'huile de chanvre, et leurs mélanges.

De préférence, l'huile H2 est choisie parmi les huiles riches en acides gras polyinsaturés.

On entend par "acide gras insaturé" au sens de la présente invention, un acide gras comprenant au moins une double liaison. Il s'agit plus particulièrement d'acides gras à longues chaînes, c'est-à-dire pouvant posséder plus de 14 atomes de carbone. Les acides gras insaturés peuvent être sous forme acide, ou sous forme de sel, comme par exemple leur sel de calcium, ou encore sous forme de dérivés, notamment d'ester(s) d'acide(s) gras.

De préférence, l'huile H2 est choisie parmi les huiles riches en acides gras à longues chaînes, c'est-à-dire pouvant posséder plus de 14 atomes de carbone, et mieux en acides gras insaturés comportant de 18 à 22 atomes de carbone, en particulier en acides gras ω-3 et ω-6. Ainsi, avantageusement, les huiles végétales sont choisies parmi les huiles d'onagre, de bourrache, de pépins de cassis, de chanvre, de noix, de soja, de tournesol, de germes de blé, de fénugrec, de rosier muscat, d'échium, d'argan, de baobab, de son de riz, de sésame, d'amande, de noisette, de chia, de lin, d'olive, d'avocat, de carthame, de coriandre, de colza (notamment Brassica naptus), et leurs mélanges.

De préférence, l'huile H2 est choisie parmi les huiles mates et non brillantes. Peut notamment être citée à ce titre l'huile de Moringa.

Selon un mode de réalisation, la teneur en huile(s) H2 dans la phase grasse est comprise entre 0% et 40%, de préférence entre 0,1% et 25%, et en particulier entre 1% et 20%, en poids par rapport au poids total de ladite phase grasse.

Selon un mode de réalisation, le ratio massique entre la quantité d'huile(s) H1 et la quantité d'huile(s) H2 va de 0,025 à 99,99, de préférence de 0,8 à 90, et en particulier de 2,5 à 80.

La phase grasse peut comprendre en outre au moins une autre huile différente des huiles H1 et H2.

Une émulsion selon l'invention de type huile-dans-eau peut comprendre de 0,0001% à 50%, de préférence de 0,1% à 40%, et mieux de 1% à 25%, en poids d'huile(s) par rapport au poids total de ladite émulsion.

Une émulsion selon l'invention de type eau-dans-huile peut comprendre de 50 % à 89,9%, de préférence de 55% à 80%, et mieux de 60% à 70%, en poids d'huile(s) par rapport au poids total de ladite émulsion.

*Corps gras solides*

Selon un mode de réalisation, les gouttes de la phase dispersée peuvent comprennent au moins un corps gras solides à température et pression ambiante choisis parmi les cires, les corps gras pâteux, les beurres, et leurs mélanges.

*Cire(s)*

Par « cire », on entend au sens de l'invention, un composé lipophile, solide à température ambiante (25°C), à changement d'état solide/liquide réversible, ayant un point de fusion supérieur ou égal à 30 °C pouvant aller jusqu'à 120°C.

Le protocole de mesure de ce point de fusion est décrit plus loin.

Les cires susceptibles d'être utilisées dans une émulsion selon l'invention peuvent être choisies parmi les cires, solides, déformables ou non à température ambiante, d'origine animale, végétale, minérale ou de synthèse et leurs mélanges.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters, et leurs mélanges.

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transestérification et hydrogénation d'huiles végétales, telles que l'huile de ricin ou d'olive, comme les cires vendues sous les dénominations de Phytowax ricin 16L64® et 22L73® et Phytowax Olive 18L57 par la société SOPHIM. De telles cires sont décrites dans la demande FR-A-2792190.

On peut aussi utiliser des cires siliconées qui peuvent être avantageusement des polysiloxanes substitués, de préférence à bas point de fusion.

Parmi les cires de silicones commerciales de ce type, on peut citer notamment celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être des alkyl ou alcoxydiméthicones tels que les produits

commerciaux suivants : Abilwax 2428, 2434 et 2440 (GOLDSCHMIDT), ou VP 1622 et VP 1621 (WACKER), ainsi que les ($C_{20}$-$C_{60}$) alkyldiméthicones, en particulier les ($C_{30}$-$C_{45}$) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

*Beurre(s) ou corps gras pâteux*

Par « beurre» (également appelé « corps gras pâteux ») au sens de la présente invention, on entend un composé gras lipophile à changement d'état solide/liquide réversible et comportant à la température de 25°C une fraction liquide et une fraction solide, et à pression atmosphérique (760 mm Hg). En d'autres termes, la température de fusion commençante du composé pâteux peut être inférieure à 25°C. La fraction liquide du composé pâteux mesurée à 25°C peut représenter de 9% à 97 % en poids du composé. Cette fraction liquide à 25°C représente de préférence entre 15% et 85 %, de préférence encore entre 40 et 85 % en poids. De préférence, le ou les beurres présentent une température de fin de fusion inférieure à 60°C. De préférence, le ou les beurres présentent une dureté inférieure ou égale à 6 MPa.

De préférence, les beurres ou corps gras pâteux présentent à l'état solide une organisation cristalline anisotrope, visible par observations aux rayons X. Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination "DSC Q2000" par la société TA Instruments.

Concernant la mesure de la température de fusion et la détermination de la température de fin de fusion, les protocoles de préparation des échantillons et de mesure sont les suivants : Un échantillon de 5 mg de corps gras pâteux (ou beurre) ou de cire préalablement chauffé à 80°C et prélevés sous agitation magnétique à l'aide d'une spatule également chauffée est placé dans une capsule hermétique en aluminium, ou creuset. Deux essais sont réalisés pour s'assurer de la reproductibilité des résultats.

Les mesures sont réalisées sur le calorimètre mentionné ci-dessus. Le four est soumis à un balayage d'azote. Le refroidissement est assuré par l'échangeur thermique RCS 90. L'échantillon est ensuite soumis au protocole suivant en étant tout d'abord mis en température à 20°C, puis soumis à une première montée en température allant de 20°C à 80°C, à la vitesse de chauffe de 5°C/minute, puis est refroidi de 80°C à -80°C à une vitesse de refroidissement de 5°C/minute et enfin soumis à une deuxième montée en température allant de -80°C à 80°C à une vitesse de chauffe de 5°C/minute. Pendant la deuxième montée en température, on mesure la variation de la différence de puissance absorbée par le creuset vide et par le creuset contenant l'échantillon de beurre en fonction de la température. Le point de fusion du composé est la valeur de la température correspondant au sommet du pic de la courbe représentant la variation de la différence de puissance absorbée en fonction de la température. La température de fin de fusion correspond à la température à laquelle 95% de l'échantillon a fondu.

La fraction liquide en poids du beurre (ou corps gras pâteux) à 25°C est égale au rapport de l'enthalpie de fusion consommée à 25°C sur l'enthalpie de fusion du beurre. L'enthalpie de fusion du beurre ou composé pâteux est l'enthalpie consommée par le composé pour passer de l'état solide à l'état liquide.

Le beurre est dit à l'état solide lorsque l'intégralité de sa masse est sous forme solide cristalline. Le beurre est dit à l'état liquide lorsque l'intégralité de sa masse est sous forme liquide. L'enthalpie de fusion du beurre est égale à l'intégrale de l'ensemble de la courbe de fusion obtenue à l'aide du calorimètre suscité, avec une montée en température de 5°C ou 10°C par minute, selon la norme ISO 11357-3:1999. L'enthalpie de fusion du beurre est la quantité d'énergie nécessaire pour faire passer le composé de l'état solide à l'état liquide. Elle est exprimée en J/g.

L'enthalpie de fusion consommée à 25°C est la quantité d'énergie absorbée par l'échantillon pour passer de l'état solide à l'état qu'il présente à 25°C constitué d'une fraction liquide et d'une fraction solide. La fraction liquide du beurre mesurée à 32°C représente de préférence de 30% à 100 % en poids du composé, de préférence de 50% à 100%, de préférence encore de 60% à 100 % en poids du composé. Lorsque la fraction liquide du beurre mesurée à 32°C est égale à 100%, la température de la fin de la plage de fusion du composé pâteux est inférieure ou égale à 32°C. La fraction liquide du beurre mesurée à 32°C est égale au rapport de l'enthalpie de fusion consommée à 32°C sur l'enthalpie de fusion du beurre. L'enthalpie de fusion consommée à 32 °C est calculée de la même façon que l'enthalpie de fusion consommée à 23 °C.

Concernant la mesure de la dureté, les protocoles de préparation des échantillons et de mesure sont les suivants : la composition selon l'invention ou le beurre est placé dans un moule de 75 mm de diamètre qui est rempli à environ 75% de sa hauteur. Afin de s'affranchir du passé thermique et de contrôler la cristallisation, le moule est placé à l'étuve programmable Vötsch VC0018 où il est tout d'abord mis en température à 80°C pendant 60 minutes, puis refroidi de 80°C à 0°C à une vitesse de refroidissement de 5°C/minute, puis laissé à la température stabilisée de 0°C pendant 60 minutes, puis soumis à une montée en température allant de 0°C à 20°C, à une vitesse de chauffe de

5°C/minute, puis laissé à la température stabilisée de 20°C pendant 180 minutes. La mesure de la force de compression est réalisée avec le texturomètre TA/TX2i de Swantech. Le mobile utilisé est choisi selon la texture : - mobile cylindrique en acier de 2 mm de diamètre pour les matières premières très rigides ; - mobile cylindrique en acier de 12 mm de diamètre pour les matières premières peu rigides. La mesure comporte 3 étapes : une 1ère étape après détection automatique de la surface de l'échantillon où le mobile se déplace à la vitesse de mesure de 0,1 mm/s, et pénètre dans la composition selon l'invention ou le beurre à une profondeur de pénétration de 0,3 mm, le logiciel note la valeur de la force maximale atteinte ; une 2ème étape dite de relaxation ou le mobile reste à cette position pendant une seconde et où on note la force après 1 seconde de relaxation ; enfin une 3ème étape dite de retrait ou le mobile revient à sa position initiale à la vitesse de 1 mm/s et on note l'énergie de retrait de la sonde (force négative).

La valeur de la dureté mesurée lors de la première étape correspond à la force de compression maximale mesurée en Newton divisée par la surface du cylindre du texturomètre exprimée en $mm^2$ en contact avec le beurre ou la composition selon l'invention. La valeur de dureté obtenue est exprimée en méga-pascals ou MPa.

Le corps gras pâteux ou beurre peut être choisi parmi les composés synthétiques et les composés d'origine végétale. Un corps gras pâteux peut être obtenu par synthèse à partir de produits de départ d'origine végétale.

Le corps gras pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters, et
- leurs mélanges.

Selon un mode préféré de l'invention le ou les beurres particuliers sont d'origine végétale tels que ceux décrit dans Ullmann's Encyclopedia of Industrial Chemistry (« Fats and Fatty Oils», A. Thomas, publié le 15/06/2000, D01: 10.1002/14356007.a10_173, point 13.2.2.2. Shea Butter, Borneo Tallow, and Related Fats (Vegetable Butters)).

On peut citer plus particulièrement les triglycérides en C10-C18 (nom INCI : C10-18 Triglycerides) comportant à la température de 25°C et à pression atmosphérique (760 mm Hg) une fraction liquide et une fraction solide, le beurre de karité, le beurre de Karité Nilotica (*Butyrospermum parkii*), le beurre de Galam, (*Butyrospermum parkii*), le beurre ou graisse de Bornéo ou tengkawang tallow) (*Shorea stenoptera),* beurre de Shorea, beurre d'Illipé , beurre de Madhuca ou Bassia Madhuca longifolia, beurre de mowrah (*Madhuca Latifolia*), beurre de Katiau (*Madhuca mottleyana),* le beurre de Phulwara (M. *butyracea*), le beurre de mangue (*Mangifera indica*), le beurre de Murumuru (*Astrocatyum murumuru*), le beurre de Kokum (*Garcinia Indica*), le beurre d'Ucuuba (*Virola sebifera*), le beurre de Tucuma, le beurre de Painya (Kpangnan) (*Pentadesma butyracea*), le beurre de café (*Coffea arabica*), le beurre d'abricot (*Prunus Armeniaca*), le beurre de Macadamia (*Macadamia Temifolia*), le beurre de pépin de raisin (*Vitis vinifera*), le beurre d'avocat *(Persea gratissima),* le beurre d'olives (*Olea europaea*), le beurre d'amande douce (*Prunus amygdalus dulcis*), le beurre de cacao (*Theobroma cacao*) et le beurre de tournesol, le beurre sous le nom INCI Astrocaryum Murumuru Seed Butter, le beurre sous le nom INCI Theobroma Grandiflorum Seed Butter, et le beurre sous le nom INCI Irvingia Gabonensis Kernel Butter, les esters de jojoba (mélange de cire et d'huile de jojoba hydrogénée)(nom INCI : Jojoba esters) et les esters éthyliques de beurre de karité (nom INCI : Shea butter ethyl esters), et leurs mélanges.

Bien entendu, l'homme du métier veillera à choisir le(s) corps gras solide(s) et/ou leur quantité de telle manière que les propriétés avantageuses de l'émulsion selon l'invention ne soient pas ou substantiellement pas altérées par l'adjonction envisagée, notamment de manière à préserver l'intégrité de l'émulsion selon l'invention et notamment de l'écorce (ou membrane) des gouttes dispersées. Ces ajustements relèvent des compétences de l'homme du métier.

De préférence, une émulsion selon l'invention peut comprendre de 0% à 98,99% en poids, de préférence de 0,5% à 70% en poids, en particulier de 1% à 30% en poids, et mieux de 1% à 20% en poids, de corps gras solide(s)

par rapport au poids total de la phase grasse.

Selon un mode de réalisation, la phase grasse a une viscosité comprise de 0,1 mPa.s à 1 000 000 mPa.s, de préférence de 0,5 mPa.s à 500 000 mPa.s, et mieux de 1 à 1000 mPa.s, telle que mesurée à 25°C. La mesure de la viscosité est typiquement réalisée selon le mode opératoire détaillé précédemment pour la mesure de la viscosité de l'émulsion.

### *Ecorce des gouttes*

Comme mentionné précédemment, les gouttes d'une émulsion selon l'invention sont entourées d'une écorce (également désignée par le terme « membrane »).

Selon l'invention, les gouttes obtenues peuvent présenter une écorce très fine, notamment d'épaisseur inférieure à 1% du diamètre des gouttes.

L'épaisseur de l'écorce est ainsi de préférence inférieure à 1 $\mu$m et est trop faible pour être mesurée par des méthodes optiques.

Selon un mode de réalisation, l'épaisseur de l'écorce des gouttes est inférieure à 1 000 nm, notamment comprise de 1 à 500 nm, de préférence inférieure à 100 nm, avantageusement inférieure à 50 nm, préférentiellement inférieure à 10 nm.

La mesure de l'épaisseur de l'écorce des gouttes de l'invention peut être effectuée par la méthode de diffusion de neutrons aux petits angles (Small-Angle X-ray Scattering), telle que mise en oeuvre dans Sato et al. J. Chem. Phys. 111, 1393-1401 (2007).

Pour cela, les gouttes sont produites en utilisant de l'eau deutérée, puis sont lavées trois fois avec une huile deutérée, comme par exemple une huile deutérée de type hydrocarboné (octane, dodécane, hexadécane).

Après lavage, les gouttes sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

Selon un mode de réalisation, l'écorce entourant les gouttes de la phase dispersée est rigidifiée, ce qui confère notamment une bonne résistance aux gouttes et diminue, voire empêche, leur coalescence.

Cette écorce est typiquement formée par coacervation, c'est-à-dire par précipitation de polymères chargés de charges opposées. Au sein d'un coacervat, les liaisons liant les polymères chargés entre eux sont de type ionique, et sont généralement plus fortes que des liaisons présentes au sein d'une membrane de type tensioactif.

L'écorce est formée par coacervation d'au moins deux polymères chargés de polarité opposée (ou polyélectrolyte) et de préférence en présence d'un premier polymère, de type cationique, et d'un deuxième polymère, différent du premier polymère, de type anionique. Ces deux polymères jouent le rôle d'agents de rigidification de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le polymère anionique et le polymère cationique. La membrane ainsi formée autour de chaque goutte forme typiquement une écorce qui encapsule totalement le coeur de la goutte et isole ainsi le coeur de la goutte de la phase continue.

### *Polymère anionique*

La phase aqueuse, notamment la phase aqueuse continue, comprend au moins un polymère anionique.

Dans le cadre de la présente description, on entend par "polymère anionique" (ou "polymère de type anionique") un polymère comportant des fonctions chimiques de type anionique. On peut aussi parler de polyélectrolyte anionique.

Par "fonction chimique de type anionique", on entend une fonction chimique AH capable de céder un proton pour donner une fonction A. Selon les conditions du milieu dans lequel il se trouve, le polymère de type anionique comporte donc des fonctions chimiques sous forme AH, ou bien sous forme de sa base conjuguée A.

Comme exemple de fonctions chimiques de type anionique, on peut citer les fonctions acides carboxyliques -COOH, éventuellement présentes sous forme d'anion carboxylate -COO-.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique. Il peut par exemple s'agir de polymère anionique comprenant des unités monomères comportant au moins une fonction chimique de type acide carboxylique.

De préférence, le polymère anionique est hydrophile, c'est-à-dire soluble ou dispersible dans l'eau. Dans le cadre de l'invention, et sauf mention contraire, on entend par « hydrophile », la propriété selon laquelle un corps donné

est compatible avec de l'eau ou un solvant polaire, c'est-à-dire peut accepter de l'eau ou ledit solvant, pour former avec eux une phase homogène, par exemple une solution.

Parmi les exemples de polymère anionique appropriés à la mise en oeuvre de l'invention, on peut citer les copolymères d'acide acrylique ou d'acide maléique et d'autres monomères, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester, les acrylates crosspolymères.

Selon un mode de réalisation, le polymère anionique selon l'invention est un carbomère ou un copolymère réticulé acrylates/$C_{10-30}$ alkyl acrylate. De préférence, le polymère anionique selon l'invention est un carbomère.

Selon un mode de réalisation, l'écorce des gouttes comprend au moins un polymère anionique, tel que par exemple un carbomère.

Dans le cadre de l'invention, et sauf mention contraire, on entend par "carbomère", un homopolymère éventuellement réticulé, issu de la polymérisation de l'acide acrylique. Il s'agit donc d'un poly(acide acrylique) éventuellement réticulé.

Parmi les carbomères de l'invention, on peut citer ceux commercialisés sous les noms Tego®Carbomer 340FD de Evonik, Carbopol® 981 de Lubrizol, Carbopol ETD 2050 de Lubrizol, ou encore Carbopol Ultrez 10 de Lubrizol.

Selon un mode de réalisation, on entend par "carbomère" ou "carbomer" ou "Carbopol®" un polymère d'acide acrylique de haut poids moléculaire réticulé avec du sucrose allylique ou des éthers allyliques de pentaérythritol (handbook of Pharmaceutical Excipients, 5eme Edition, plll). Par exemple, il s'agit du Carbopol®910, du Carbopol®934, Carbopol®934P, du Carbopol®940, du Carbopol®941, du Carbopol®71G, du Carbopol®980, du Carbopol®971P ou du Carbopol®974P. Selon un mode de réalisation, la viscosité dudit carbomère est comprise entre 4 000 et 60 000 cP à 0,5% w/w.

Les carbomères ont d'autres dénominations : acides polyacryliques, polymères carboxyvinyliques ou carboxy polyéthylènes.

Une émulsion selon l'invention peut comprendre de 0,01% à 5%, de préférence de 0,05% à 2%, et préférentiellement de 0,10% à 0,5%, en poids de polymère(s) anionique(s), notamment de carbomère(s), par rapport au poids total de ladite émulsion.

Selon l'invention, le polymère anionique peut également être un copolymère réticulé acrylates/$C_{10-30}$ alkyl acrylate (nom INCI : acrylates/$C_{10-30}$ alkyl acrylate Crosspolymer) tel que défini ci-dessus.

Selon l'invention, les émulsions selon l'invention peuvent comprendre un carbomère et un copolymère réticulé acrylates/$C_{10-30}$ alkyl acrylate.

La phase aqueuse selon l'invention peut également comprendre au moins un polymère réticulé ou au moins un copolymère réticulé, ledit polymère réticulé ou copolymère réticulé comprenant au moins une unité dérivée de la polymérisation d'un des monomères suivants : acide acrylique ou méthacrylique, acrylate ou méthacrylate d'alkyle comprenant de 1 à 30 atomes de carbone, ou leurs sels.

La phase aqueuse peut également comprendre un mélange de polymères réticulés ou un mélange de copolymères réticulés ou encore un mélange de polymère(s) réticulé(s) et de copolymère(s) réticulé(s).

Selon l'invention, le terme "unité dérivée de la polymérisation d'un monomère" signifie que le polymère ou copolymère est un polymère ou copolymère obtenu par polymérisation ou copolymère dudit monomère.

Selon un mode de réalisation, le polymère réticulé ou le copolymère réticulé est un polyacrylate réticulé.

Les copolymères et polymères réticulés de l'invention sont anioniques.

Selon un mode de réalisation, le copolymère est un copolymère d'acide carboxylique insaturé et de carboxylate insaturé d'alkyle en $C_{1-30}$, de préférence en $C_1$-$C_4$. Un tel copolymère comporte au moins un motif hydrophile de type acide carboxylique insaturé oléfinique et au moins un motif hydrophobe de type ester d'alkyle ($C_1$-$C_{30}$) d'acide carboxylique insaturé.

De préférence, ces copolymères sont choisis parmi ceux dont le motif hydrophile de type acide carboxylique insaturé oléfinique correspond au monomère de formule (I) suivante :

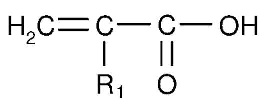
(I)

dans laquelle : $R_1$ désigne H ou $CH_3$ ou $C_2H_5$, c'est-à-dire des motifs acide acrylique, acide méthacrylique ou acide éthacrylique,

et dont le motif hydrophobe de type ester d'alkyle ($C_1$-$C_{30}$) d'acide carboxylique insaturé correspond au monomère de formule (II) suivante :

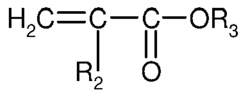 (II)

dans laquelle : $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_1$-$C_{30}$, et de préférence en $C_1$-$C_4$.

Parmi ce type de copolymères, on utilisera plus particulièrement ceux formés à partir d'un mélange de monomères comprenant :

(i) essentiellement de l'acide acrylique,
(ii) un ester de formule (II) décrite ci-dessus et dans laquelle $R_2$ désigne H ou $CH_3$, $R_3$ désignant un radical alkyle ayant de 1 à 4 atomes de carbone,
(iii) et un agent réticulant, qui est un monomère insaturé polyéthylénique copolymérisable bien connu, comme le phtalate de diallyle, le triméthylolpropane tri(meth)acrylate, diallyl itaconate, diallyl fumarate, diallyl maléate, zinc (meth)acrylate, le (méth)acrylate d'allyle, le divinylbenzène, le diméthacrylate de (poly)éthylèneglycol, le méthylène-bis-acrylamide, et l'huile de ricin.

Selon un mode de réalisation, le polymère réticulé ou le copolymère réticulé est un polymère ou copolymère d'acide acrylique et/ou d'acide méthacrylique, et/ou d'acrylate d'alkyle comprenant de 1 à 30 atomes de carbone, de préférence de 1 à 4 atomes de carbone, et/ou de méthacrylate d'alkyle comprenant de 1 à 30 atomes de carbone, de préférence de 1 à 4 atomes de carbone.

Selon un mode de réalisation, le copolymère réticulé est un copolymère réticulé d'acide méthacrylique et d'acrylate d'alkyle comprenant de 1 à 4 atomes de carbone, de préférence 2 atomes de carbone.

Dans le cadre de l'invention, et sauf mention contraire, on entend par « copolymère réticulé d'acide méthacrylique et d'acrylate d'alkyle comprenant de 1 à 4 atomes de carbones », un copolymère réticulé résultant de la polymérisation d'un monomère d'acide méthacrylique et d'un monomère d'acrylate d'alkyle comprenant de 1 à 4 atomes de carbones.

De préférence, dans ce copolymère, l'acide méthacrylique représente de 20% à 80% en poids, de préférence de 35% à 65% en poids du poids total du copolymère.

De préférence, dans ce copolymère, l'acrylate d'alkyle représente de 15% à 80% en poids, de préférence de 35% à 65% en poids du poids total du copolymère.

En particulier, l'acrylate d'alkyle est choisi parmi le méthacrylate d'alkyle, l'acrylate d'éthyle et l'acrylate de butyle.

Selon un mode de réalisation, le polymère réticulé ou le copolymère réticulé selon l'invention, présent dans la phase aqueuse, est choisi dans le groupe constitué des polymères ou copolymères suivants : Acrylates Copolymer, Acrylates crosspolymer-4, Acrylates crosspolymer-3, Polyacrylate-2 Crosspolymer et Polyacrylate-14 (noms INCI).

Parmi lesdits polymères ci-dessus, on préfère tout particulièrement, selon la présente invention, les produits vendus par la société LUBRIZOL sous les dénominations commerciales Fixate Superhold (nom INCI = Polyacrylate-2 Crosspolymer), Fixate Freestyle Polymer (nom INCI = Acrylates crosspolymer-3), Carbopol® Aqua SF1 (nom INCI = Acrylates copolymer) et Carbopol® Aqua SF2 (nom INCI = Acrylates crosspolymer-4).

De préférence, le copolymère réticulé est le Carbopol® Aqua SF1 (nom INCI = Acrylates copolymer).

Selon un mode de réalisation, le copolymère réticulé est choisi parmi les copolymères réticulés d'acide acrylique ou méthacrylique et d'acrylates d'alkyle comprenant de 1 à 4 atomes de carbone.

Selon l'invention, une émulsion selon l'invention peut comprendre de 0,1% à 10% en poids, de préférence de 0,5% à 8% en poids, et préférentiellement de 1% à 3% en poids de polymère réticulé ou copolymère réticulé par rapport au poids total de ladite émulsion.

Les émulsions selon l'invention peuvent comprendre un carbomère et un copolymère réticulé Carbopol® Aqua SF1 (nom INCI = Acrylates copolymer).

***Polymère cationique***

La phase grasse, notamment la phase grasse dispersée, comprend au moins un polymère cationique.

Selon un mode de réalisation, les gouttes, et notamment l'écorce desdites gouttes, comprennent en outre un polymère de type cationique. Elles peuvent également comprendre plusieurs polymères de type cationique. Ce polymère cationique est celui mentionné ci-dessus qui forme l'écorce par coacervation avec le polymère anionique.

Dans le cadre de la présente demande, et sauf mention contraire, on entend par "polymère cationique" (ou "polymère de type cationique") un polymère comportant des fonctions chimiques de type cationique. On peut aussi

parler de polyélectrolyte cationique.

De préférence, le polymère cationique est lipophile ou liposoluble.

Dans le cadre de la présente demande, et sauf mention contraire, par "fonction chimique de type cationique", on entend une fonction chimique B capable de capter un proton pour donner une fonction $BH^+$. Selon les conditions du milieu dans lequel il se trouve, le polymère de type cationique comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Comme exemple de polymère cationique, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymères cationiques appropriés à la mise en oeuvre de l'invention, on peut citer l'amodiméthicone, dérivé d'un polymère silicone (polydiméthylsiloxane, aussi appelé diméthicone), modifié par des fonctions amines primaire et amine secondaire.

On peut également citer des dérivés de l'amodiméthicone, comme par exemple des copolymères de l'amodiméthicone, l'aminopropyl diméthicone, et plus généralement des polymères silicones linéaires ou ramifiés comportant des fonctions amines.

On peut citer le copolymère de bis-isobutyl PEG-14/amodiméthicone, le Bis (C13-15 Alkoxy) PG-Amodimethicone, le Bis-Cetearyl Amodimethicone et le bis-hydroxy/méthoxy amodiméthicone.

On peut également citer les polymères de type polysaccharide comprenant des fonctions amines, tel que le chitosan ou les dérivés de gomme guar (chlorure d'hydroxypropyltrimonium guar).

On peut également citer les polymères de type polypeptide comprenant des fonctions amines, tel que la polylysine.

On peut également citer les polymères de type polyéthylèneimine comprenant des fonctions amines, tel que la polyéthylèneimine linéaire ou branchée.

Selon l'invention, les gouttes, et notamment l'écorce desdites gouttes, comprennent un polymère cationique qui est un polymère silicone modifié par une fonction amine primaire, secondaire ou tertiaire, tel que l'amodiméthicone.

Selon un mode de réalisation, les gouttes, et en particulier l'écorce desdites gouttes, comprennent de l'amodiméthicone.

Selon un mode de réalisation préféré, l'amodiméthicone ne contient pas de cyclométhicone, tel que par exemple le cyclotétrasiloxane ou le cyclopentasiloxane.

Selon un mode de réalisation, la viscosité du polymère cationique, et notamment de l'amodiméthicone, est comprise de 200 mPa.s à 50 000 mPa.s, de préférence de 500 mPa.s à 10 000 mPa.s, et encore plus préférentiellement de 800 à 4 000 mPa.s telle que mesurée à 25°C.

Selon un mode de réalisation particulièrement préféré, le polymère cationique répond à la formule (I) suivante : dans laquelle :

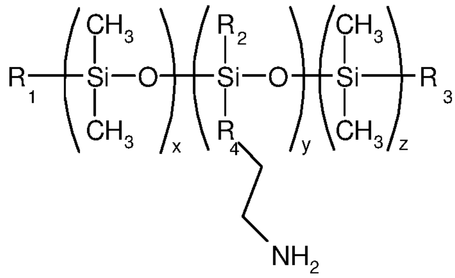

(I)

- $R_1$, $R_2$ et $R_3$, indépendamment les uns des autres, représentent OH ou $CH_3$ ;
- $R_4$ représente un groupe $-CH_2-$ ou un groupe -X-NH- dans lequel X est un radical alkylène divalent en C3 ou C4 ;
- x est un nombre entier compris entre 10 et 5 000, de préférence entre 30 et 1 000, et mieux entre 80 et 300 ;
- y est un nombre entier compris entre 2 et 1 000, de préférence entre 4 et 100, et mieux entre 5 et 20 ; et
- z est un nombre entier compris entre 0 et 10, de préférence entre 0 et 1, et mieux est égal à 1.

Dans la formule (I) susmentionnée, lorsque $R_4$ représente un groupe -X-NH-, X est relié à l'atome de silicium.

Dans la formule (I) susmentionnée, $R_1$, $R_2$ et $R_3$ représentent de préférence $CH_3$.

Dans la formule (I) susmentionnée, $R_4$ est de préférence un groupe $-(CH_2)_3-NH-$,

Selon un mode de réalisation particulièrement préféré, le polymère cationique répond à la formule (I-1) suivante : dans laquelle :

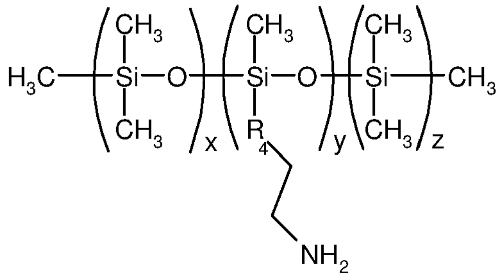

(I-1)

- $R_4$ est tel que défini précédemment, et est de préférence un groupe $-(CH_2)_3-NH-$ ;
- x est un nombre entier compris entre 80 et 300, de préférence entre 100 et 200 ;
- y est un nombre entier compris entre 5 et 20, de préférence entre 5 et 15 ; et
- z est un nombre entier compris entre 0 et 1, et de préférence égal à 1.

Selon un mode de réalisation, dans la formule (I-1) susmentionnée, $R_4$ est un groupe $-(CH_2)_3-NH-$, z égal à 1, x est compris entre 80 et 300, et y est compris entre 5 et 15.

Selon un mode de réalisation, dans la formule (I-1) susmentionnée, $R_4$ un groupe $-(CH_2)_3-NH-$, z égal à 1, x est compris entre 100 et 200, et y est compris entre 5 et 15.

Selon un mode de réalisation, dans la formule (I-1) susmentionnée, $R_4$ un groupe $-(CH_2)_3-NH-$, z égal à 1, x est compris entre 100 et 150, et y est compris entre 5 et 12.

Selon un mode de réalisation, la quantité de fonctions amines présente dans (ou portée/apportée par) le polymère cationique, et notamment dans l'amodiméthicone, est comprise entre 0,01 et 12,3, de préférence entre 0,1 et 8, plus préférentiellement entre 0,5 et 4, mmol, voire entre 0,5 et 2 mmol, par gramme de polymère cationique.

Dans le cadre de l'invention, et sauf mention contraire, on entend par « la quantité de fonctions amines présentes dans (ou apportée par) le polymère cationique », la quantité de fonctions amines portées par le polymère cationique.

Pour des raisons évidentes, les fonctions amines apportées par le polymère cationique considérées sont celles aptes à réagir avec le polymère anionique, en particulier avec les groupes (ou fonctions) carboxyliques portés par ledit polymère anionique. Il s'agit de préférence des fonctions amines présentes sur les chaînes ramifiées du polymère cationique.

Ainsi, avantageusement, au moins 50%, de préférence au moins 60%, en particulier au moins 70%, mieux au moins 80%, de préférence au moins 90%, et tout particulièrement au moins 99%, des fonctions amines portées par le polymère cationique sont aptes à réagir avec le polymère anionique, en particulier avec les groupes carboxyliques portés par ledit polymère anionique.

Afin d'assurer un pontage efficace entre les fonctions amines du polymère cationique et la/les fonction(s) carboxylique(s) du polymère anionique, et comme indiqué précédemment, un polymère cationique selon l'invention comprend au moins deux fonctions amines. A cet égard et de préférence, un polymère cationique selon l'invention comprend au moins deux fonctions amines localisées sur des chaines ramifiées différentes dudit polymère cationique. En d'autres termes, un polymère cationique selon l'invention comprend au moins deux chaines ramifiées, identiques ou différentes, chaque chaine ramifiée comprenant au moins une fonction amine apte à réagir avec le polymère anionique, en particulier avec au moins un groupe carboxylique porté par ledit polymère anionique.

Selon un mode de réalisation, le polymère cationique, et notamment l'amodiméthicone, présente une viscosité comprise de 200 mPa.s à 50 000 mPa.s et/ou la quantité de fonctions amines présentes dans ledit polymère cationique est comprise entre 0,01 et 12,3, de préférence entre 0,1 et 10,0, mieux entre 0,5 et 4,0 et en particulier entre 1,0 et 3,0 mmol par gramme dudit polymère cationique.

De préférence, le polymère cationique, et notamment l'amodiméthicone, présente une viscosité comprise de 700 mPa.s à 4 000 mPa.s et/ou la quantité de fonctions amines présentes dans ledit polymère cationique est comprise entre 0,5 et 3,0 mmol par gramme dudit polymère cationique.

*Méthode de détermination de la quantité en fonctions amines présente dans le polymère cationique, notamment l'amodiméthicone*

La quantité de fonctions amines présentes dans le polymère cationique peut notamment être mesurée selon la méthode suivante :

**Paramètres**

Sauf indication contraire, effectuer le test décrit ci-après en conditions ambiantes, à savoir à température ambiante (25 ° C) et à 30% - 70% d'humidité relative.
Sécher du tris(hydroxyméthyl)aminométhane (TRIS) (No CAS 77-86-1) à 110 ° C pendant 2 heures minimum et refroidir sur une semaine.

**Méthode**

***Préparation de la solution de solvant (i.e. solution A)***

Mesurer 1000 ml de toluène de qualité HPLC dans une bouteille de couleur ambrée.
Ajouter 10 ml d'eau distillée.
Ajouter 990 ml d'alcool isopropylique.
Fermer la bouteille et mélanger doucement la solution pendant environ une minute.
On obtient ainsi la solution A.
Vérifier que la solution A est légèrement acide (pH ~ 5) en ajoutant 3 gouttes d'une solution d'indicateur pourpre de bromocrésol à une aliquote de 100 ml de la solution A.
Agiter pendant environ 30 secondes.
La solution de solvant devrait apparaître jaune / vert après mélange.

***Préparation de 0,5% d'une solution d'indicateur vert de Bromocrésol (i.e. solution B)***

Mesurer dans une bouteille 0,50 ± 0,01 grammes d'un colorant vert de bromocrésol.
Ajouter 100 ml d'éthanol.
Fermer la bouteille et agiter vigoureusement.
On obtient ainsi la solution B.

**Titrage d'une solution d'HCl (i.e. solution C)**

Mesurer une quantité appropriée en TRIS. Enregistrer la masse de TRIS **M1.**
NOTE : avec de l'HCl 0,1 N, ajouter environ 0,2 grammes de TRIS séché; avec de l'HCl 1 N, ajouter environ 1,8 grammes de TRIS séché.
Ajouter 100 ml d'eau déminéralisée.
Ajouter 3 gouttes de solution B.
Agiter pendant environ 3 minutes.
REMARQUE: la solution de TRIS devrait apparaître bleue après agitation.
Positionner une burette de 25 ml.
Remplir la burette d'une solution standard d'HCl. Purger le haut de la burette de l'air en permettant à environ 1,0 ml **d'HCl** de s'écouler à travers le haut de la burette.
La lecture de la solution standard **d'HCl** dans la burette doit être comprise entre 0,00 ~ 1,00 ml.
Enregistrer le volume **d'HCl** dans la burette **V1.**
Commencer le titrage en ajoutant la solution standard **d'HCl** par addition de 0,5 ml à la solution TRIS tout en agitant ladite solution TRIS avec un agitateur magnétique.
Approcher du point final de la titration avec prudence en ajoutant lentement de petites gouttes de solution standard **d'HCl** à la solution TRIS jusqu'à ce que la couleur de la solution TRIS change du bleu au jaune et reste jaune pendant au moins 60 secondes.
NOTE: La couleur de la solution TRIS peut changer temporairement du bleu au vert à proximité de l'endroit où le titrant est ajouté. Comme produit de titrage, le changement de couleur de la solution TRIS deviendra plus dispersé et durera plus longtemps. Le titrage est terminé lorsque couleur de la solution TRIS vire au jaune par ajout de la dernière goutte de réactif et le changement de couleur persiste pendant au moins 60 secondes.
Enregistrer le volume final **d'HCl** dans la burette **V2.**
Répétez jusqu'à ce que trois titrages réussis soient achevés.

***Titration du polymère cationique***

Mesurer 0,05 à 5 grammes du polymère cationique considéré, notamment d'amodiméthicone, en fonction de la teneur en amine dans ledit polymère cationique.

Enregistrer la masse du polymère cationique **M2.**

Ajouter 50 ml de la solution A.

NOTE: Si le polymère cationique ne se dissout pas dans la solution A, ajouter d'abord l'isopropanol puis le toluène et l'eau en rapport approprié.

Ajouter 3 gouttes de la solution B.

On obtient ainsi la solution D.

Mélanger la solution D pendant 5 minutes minimum ou jusqu'à ce que la solution D semble homogène.

REMARQUE: la solution D devrait passer du bleu au vert après agitation.

Positionner une burette de 25 ml.

Remplir la burette d'une solution standard d'HCl. Purger le haut de la burette de l'air en permettant à environ 1,0 ml **d'HCl** de s'écouler à travers le haut de la burette.

La lecture de la solution standard **d'HCl** dans la burette doit être comprise entre 0,00 ~ 1,00 ml.

Enregistrer le volume de la solution standard d'HCl dans la burette **V3.**

Commencez le titrage en ajoutant à la solution D ci-dessus la solution standard d'HCl par additions de 0,5 ml tout en agitant la solution D avec un agitateur magnétique.

Approcher du point final de titration avec prudence en ajoutant lentement de petites gouttes de solution standard d'HCl jusqu'à ce que la couleur de la solution D passe du bleu au jaune et reste jaune pendant au moins 60 secondes.

NOTE: La couleur de la solution D va changer temporairement du bleu au jaune ou vert à proximité de l'endroit où le titrant est ajouté. Dans certains échantillons, la couleur peut changer du bleu au violet au jaune. Le titrage est terminé lorsque la couleur de la solution D vire au jaune en ajoutant dernière goutte de réactif et le changement de couleur persiste pendant au moins 60 secondes.

Enregistrer le volume final de solution standard d'HCl dans la burette comme **V4.**

**Calculs**

Calcul de la normalité de la solution standard de l'acide chlorhydrique :

$$N = \frac{M_1}{(0.12114)(V_2 - V_1)}$$

N = normalité de l'HCl, en N
M1 = masse du TRIS, en grammes
V1 = volume initial de solution standard d'HCl dans la burette, en mL
V2 = volume final de solution standard d'HCl dans la burette, en mL

Calcul de la teneur en groupes amine (en millimole d'amine / gramme de polymère cationique)

$$Amine = \frac{N \times (V_4 - V_3)}{M_2}$$

M2 = masse du polymère cationique, en grammes
V3 = volume initial de solution standard d'HCl dans la burette, en mL
V4 = volume final de solution standard d'HCl dans la burette, en mL
N = signifie la normalité de la solution étalon d'HCl dans la burette, en N

**Formules**

Calcul de la normalité de la solution standard d'HCl (N):

$$N(eq/L) = (gram\ de\ TRIS)\left(\frac{1\ mole\ TRIS}{121.135\ g\ TRIS}\right) \times \left(\frac{1\ eq\ TRIS}{1\ mole\ TRIS}\right) \times \left(\frac{1\ eq\ HCl}{1\ eq\ TRIS}\right) \times \left(\frac{1}{mL\ HCl}\right) \times \left(\frac{1000\ mL}{1\ L}\right)$$

Calcul de la teneur en amine (en millimole / gramme de polymère cationique):

$$\text{Amine millimole/gram} = (mL\ de\ HCl) \times \left(\frac{eq\ d'HCl}{L\ d'HCl}\right) \times \left(\frac{1}{gram}\right) \times \left(\frac{1\ mole\ Amine}{1\ eq\ d'HCl}\right) \times \left(\frac{1\ L\ HCl}{1000\ mL\ HCl}\right) \times \left(\frac{1000\ mmole\ Amine}{1\ mole\ amine}\right)$$

*Méthode de détermination de la quantité en fonctions amines apportées par le polymère cationique, notamment l'amodiméthicone, dans la phase grasse*

Partant d'un polymère cationique spécifique, l'homme du métier est en mesure de procéder aux calculs appropriés pour déterminer la quantité requise en polymère cationique considéré pour satisfaire aux exigences en termes de quantité de fonctions amines apportées par le polymère cationique dans la phase grasse telle que visée dans la présente invention.

On peut à ce titre citer la méthode de calcul suivante :

$$Q = A \times T$$

où:

- Q représente la quantité de fonctions amines apportées par le polymère cationique dans la phase grasse (en μmol/g),

  A représente le pourcentage massique du polymère cationique considéré dans la phase grasse, et

  T représente le taux de fonctions amines portées par ledit polymère cationique considéré (en μmol/g de polymère cationique), notamment obtenu par la méthode décrite ci-dessus.

Le polymère cationique selon l'invention peut être l'un des produits commerciaux suivants : CAS 3131 de Nusil, KF 8005 S ou KF 8004 de Shin Etsu, Silsoft AX ou SF 1708 de Momentive et DC 8500, DC 2-2078 ou DC 2-8566 de Dow Corning.

Le polymère cationique selon l'invention peut être une amodiméthicone tel que, par exemple, l'un des produits commerciaux suivants : CAS 3131 de Nusil, KF 8005 S ou KF 8004 de Shin Etsu, SF 1708 de Momentive et DC 2-8566 de Dow Corning.

Selon un mode de réalisation, une amodiméthicone selon l'invention est distincte/différente d'une huile telle que celles décrites précédemment et susceptibles de composer la phase grasse des gouttes d'une émulsion selon l'invention.

Avantageusement, chaque goutte d'une émulsion selon l'invention peut comprendre de 0,01% à 10%, de préférence de 0,05% à 5% en poids de polymère(s) cationique(s), notamment d'amodiméthicone(s), par rapport au poids total de la phase grasse.

Selon l'invention, l'émulsion de l'invention peut comprendre de 0,01% à 5%, de préférence de 0,01% à 2%, et préférentiellement de 0,02% à 0,5% en poids de polymère(s) cationique(s), notamment d'amodiméthicone(s), par rapport au poids total de ladite émulsion. En particulier, l'émulsion susmentionnée peut comprendre de 0,020% à 0,1% en poids de polymère(s) cationique(s) par rapport au poids total de ladite émulsion.

***Phase aqueuse***

Une émulsion selon l'invention comprend une phase aqueuse, notamment une phase aqueuse continue, de préférence sous forme d'un gel.

Outre le polymère anionique tel que défini ci-dessus, la phase aqueuse des émulsions selon l'invention comprend de l'eau.

Outre l'eau distillée ou déionisée, une eau convenant à l'invention peut être aussi une eau de source naturelle ou une eau florale.

Selon un mode de réalisation, le pourcentage massique d'eau de la phase aqueuse, notamment de la phase aqueuse continue, est d'au moins 40%, et mieux au moins 50%, notamment compris entre 70% et 98%, préférentiellement compris entre 75% et 95%, par rapport à la masse totale de ladite phase aqueuse.

Une émulsion selon l'invention de type huile-dans-eau peut comprendre au moins 20%, de préférence au moins 30%, en particulier au moins 40%, et mieux au moins 50% en poids d'eau par rapport au poids total de ladite émulsion.

Une émulsion selon l'invention de type eau-dans-huile peut comprendre moins de 50%, de préférence moins de 40%, en particulier moins de 30%, mieux moins de 20%, voire moins de 10%, en poids d'eau par rapport au poids

total de ladite émulsion.

De préférence, les émulsions selon l'invention de type huile-dans-eau comprennent au moins 75% en poids de phase aqueuse.

La phase continue aqueuse de l'émulsion selon l'invention peut en outre comprendre au moins une base. Elle peut comprendre une base unique ou un mélange de plusieurs bases différentes. La présence d'au moins une base dans ladite phase continue aqueuse contribue notamment à rehausser la viscosité de cette dernière.

Selon un mode de réalisation, la base présente dans la phase continue aqueuse est une base minérale.

Selon un mode de réalisation, la base minérale est choisie dans le groupe constitué des hydroxydes des métaux alcalins et des hydroxydes des métaux alcalino-terreux.

De préférence, la base minérale est un hydroxyde de métaux alcalins, et notamment NaOH.

Selon un mode de réalisation, la base présente dans la phase continue aqueuse est une base organique. Parmi les bases organiques, on peut citer par exemple l'ammoniaque, la pyridine, la triéthanolamine, l'aminométhylpropanol, ou encore la triéthylamine.

Une émulsion de type huile-dans-eau selon l'invention peut comprendre de 0,01% à 10% en poids, de préférence de 0,01% à 5% en poids, et préférentiellement de 0,02% à 1% en poids de base, de préférence de base minérale, et notamment de NaOH, par rapport au poids total de ladite émulsion.

***Gélifiant(s)***

Selon la fluidité de l'émulsion que l'on souhaite obtenir, on peut incorporer dans l'émulsion selon l'invention un ou plusieurs gélifiants différents des polymères cationique et anionique décrits précédemment.

Ainsi, dans une émulsion selon l'invention, la phase aqueuse peut comprendre au moins un gélifiant et/ou la phase grasse peut comprendre un gélifiant, différent(s) du polymère anionique et du polymère cationique.

De préférence, la phase aqueuse, notamment continue, peut comprendre à titre de gélifiant au moins un copolymère réticulé tel que décrit ci-dessus, et en particulier le Carbopol® Aqua SF1 (nom INCI = Acrylates copolymer).

***Composé(s) additionnel(s)***

Les émulsions de l'invention peuvent en outre comprendre des poudres, des paillettes, des colorants, des conservateurs, des humectants, des stabilisateurs, des chélateurs, des émollients etc... ou tout additif cosmétique usuel, et leurs mélanges.

Les émulsions selon l'invention peuvent encore comprendre au moins un actif, de préférence choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents anti-âge, les agents parfumant, et leurs mélanges.

Selon un mode de réalisation, une émulsion selon l'invention est dénuée d'agent parfumant.

Bien entendu, l'homme du métier veillera à choisir le(s) composé(s) additionnel(s) et/ou leur quantité en fonction de de la nature aqueuse ou grasse de la phase considérée et/ou de telle manière que (i) les propriétés avantageuses d'une émulsion selon l'invention et (ii) l'intégrité des gouttes formant ladite émulsion ne soient pas ou substantiellement pas altérées par l'adjonction envisagée. Ces ajustements relèvent des compétences de l'homme du métier.

Selon un mode de réalisation, les émulsions de l'invention, en particulier de type huile-dans-eau, comprennent de la glycérine. De préférence, les émulsions de l'invention comprennent au moins 5% en poids de glycérine par rapport au poids total desdites émulsions. En effet, au-delà de la texture, les émulsions selon l'invention apportent un autre avantage par rapport aux émulsions « classiques » car elles permettent d'utiliser de la glycérine, qui plus est dans des teneurs élevées.

Elles peuvent en particulier comprendre de la glycérine en une teneur supérieure ou égale à 10%, supérieure ou égale à 20%, supérieure ou égale à 30%, supérieure ou égale à 40%, voire jusqu'à 50%, en poids, par rapport au poids total de ladite émulsion.

Pour des raisons évidentes, la glycérine est présente au niveau de la phase aqueuse d'une émulsion selon l'invention.

***Procédé de préparation***

Les émulsions selon l'invention peuvent être préparées par différents procédés.

Ainsi, les émulsions selon l'invention présentent l'avantage de pouvoir être préparées selon un procédé simple « non-microfluidique », à savoir par simple émulsification.

Comme dans une émulsion classique, une solution aqueuse et une solution grasse sont préparées séparément.

C'est l'ajout sous agitation de la phase grasse dans la phase aqueuse qui crée l'émulsion directe.

Les émulsions selon l'invention peuvent également être préparées selon un procédé microfluidique, notamment comme décrit dans les demandes internationales WO 2012/120043 ou WO 2015/055748.

Selon ce mode de réalisation, les gouttes obtenues par ce procédé microfluidique présentent une distribution de taille uniforme. De préférence, la phase dispersée de l'invention est constituée d'une population de gouttes monodispersées, notamment telles qu'elles possèdent un diamètre moyen $\overline{D}$ compris de 500 $\mu$m à 3000 $\mu$m et un coefficient de variation Cv inférieur à 10%, voire inférieur à 3%.

Dans le cadre de la présente description, on entend par "gouttes monodispersées" le fait que la population de gouttes de la phase dispersée selon l'invention possède une distribution de taille uniforme. Des gouttes monodispersées présentent une bonne monodispersité. A l'inverse, des gouttes présentant une mauvaise monodispersité sont dites "polydispersées".

Selon un mode, le diamètre moyen $\overline{D}$ des gouttes est par exemple mesuré par analyse d'une photographie d'un lot constitué de N gouttes, par un logiciel de traitement d'image (Image J). Typiquement, selon cette méthode, le diamètre est mesuré en pixel, puis rapporté en $\mu$m, en fonction de la dimension du récipient contenant les gouttes de la dispersion.

De préférence, la valeur de N est choisie supérieure ou égale à 30, de sorte que cette analyse reflète de manière statistiquement significative la distribution de diamètres des gouttes de ladite émulsion.

On mesure le diamètre Di de chaque goutte, puis on obtient le diamètre moyen $\overline{D}$ en calculant la moyenne arithmétique de ces valeurs :

$$\overline{D} = \frac{1}{N}\sum_{i=1}^{N} D_i$$

A partir de ces valeurs $D_i$, on peut également obtenir l'écart-type $\sigma$ des diamètres des gouttes de la dispersion :

$$\sigma = \sqrt{\frac{\sum_{i=1}^{N}\left(D_i - \overline{D}\right)^2}{N}}$$

L'écart-type $\sigma$ d'une dispersion reflète la répartition des diamètres $D_i$ des gouttes de la dispersion autour du diamètre moyen $\overline{D}$.

En connaissant le diamètre moyen $\overline{D}$ et l'écart-type $\sigma$ d'une dispersion, on peut déterminer que l'on trouve 95,4% de la population de gouttes dans l'intervalle de diamètres [$\overline{D}$ - 2$\sigma$; $\overline{D}$ + 2$\sigma$] et que l'on trouve 68,2% de la population dans l'intervalle [$\overline{D}$ - $\sigma$; $\overline{D}$ + $\sigma$].

Pour caractériser la monodispersité de la dispersion selon ce mode de l'invention, on peut calculer le coefficient de variation :

$$C_v = \frac{\sigma}{\overline{D}}$$

Ce paramètre reflète la répartition des diamètres des gouttes en fonction du diamètre moyen de celles-ci.

Le coefficient de variation Cv des diamètres des gouttes selon ce mode de l'invention est inférieur à 10%, de préférence inférieur à 5%, voire inférieur à 3%.

Alternativement, la monodispersité peut être mise en évidence en plaçant un échantillon d'émulsion dans un flacon à section circulaire constante. Une agitation douce par rotation de un quart de tour sur une demi-seconde autour de l'axe de symétrie traversant le flacon, suivie d'un repos d'une demi-seconde est effectuée, avant de répéter l'opération en sens inverse, et ce quatre fois de suite.

Les gouttes de la phase dispersée s'organisent sous une forme cristalline lorsqu'elles sont monodispersées. Ainsi, elles présentent un empilement suivant un motif se répétant dans les trois dimensions. Il est alors possible d'observer, un empilement régulier qui indique une bonne monodispersité, un empilement irrégulier traduisant la polydispersité de la dispersion.

La présence, dans la phase grasse, de corps gras solide(s) à température et pression ambiante, telle qu'envisagée précédemment, peut nécessiter des ajustements au niveau du procédé de préparation d'une émulsion selon

l'invention. En particulier, le procédé de préparation d'une telle émulsion selon l'invention peut comprendre une étape de chauffage (entre 40°C et 150°C, notamment entre 50°C et 90°C) de la phase grasse avant mélange/mise en contact de ladite phase grasse avec la phase aqueuse et, le cas échéant et dans le cas d'un procédé « non-microfluidique » tel que susmentionné, le maintien de ce chauffage lors de l'agitation jusqu'à l'obtention de l'émulsion recherchée.

Ces ajustements relèvent des compétences générales de l'homme du métier.

Dans le cas d'une émulsion de type huile-dans-eau, les solutions (ou fluides) utilisés pour constituer la phase aqueuse continue et la phase grasse dispersée sont respectivement désignés Fluide Externe (FE) et Fluide Interne (FI).

Au vu de ce qui précède, le fluide FI comprend au moins un premier polymère précurseur du coacervat, notamment un polymère cationique, et en particulier l'amodiméthicone et au moins une huile et/ou au moins un corps gras solide à température et pression ambiante notamment tels que définis précédemment, et en outre, de façon optionnelle, au moins un composé additionnel susmentionné.

Le fluide FE comprend au moins de l'eau et au moins un deuxième polymère précurseur du coacervat, différent du premier polymère précurseur du coacervat, notamment un polymère anionique, et en particulier le carbomère, et en outre, de façon optionnelle, au moins composé additionnel susmentionné, voire une base, des conservateurs et/ou d'autres produits solubles dans l'eau tels que la glycérine.

Selon un mode de réalisation, le procédé de préparation d'une émulsion selon l'invention de type huile-dans-eau comprend une étape de formation des gouttes comprenant :

- la mise en contact d'un fluide FE et d'un fluide FI tels que définis ci-dessus ; et
- la formation des gouttes de phase grasse, constituée du fluide FI, dispersée dans une phase aqueuse continue constituée de fluide FE, lesdites gouttes comprenant une écorce isolant le coeur des gouttes de la phase grasse de la dispersion.

Selon un mode de réalisation où l'émulsion est préparée selon un procédé microfluidique, l'étape de mise en contact du fluide FE et du fluide FI tels que définis ci-dessus peut en outre comprendre la présence d'un fluide intermédiaire miscible avec le fluide FI, comme décrit dans WO 2012/120043. Ce fluide intermédiaire est destiné à former une pellicule autour de la goutte formée par le fluide FI dans le fluide FE. Ainsi, le fluide intermédiaire retarde la diffusion du premier polymère précurseur du coacervat présent dans le fluide FI jusqu'à ce que le fluide intermédiaire se soit mélangé avec le fluide FI et assure ainsi la formation de gouttes très stables stabilisées par une écorce très fine sans obstruction du dispositif microfluidique.

Selon un mode de réalisation, l'étape de formation des gouttes peut en outre comprendre une étape d'injection d'une solution d'augmentation de la viscosité de la phase aqueuse continue du fluide FE. De préférence, la solution d'augmentation de la viscosité est aqueuse. Cette solution d'augmentation de la viscosité est typiquement injectée dans le fluide externe aqueux FE après formation de la dispersion selon l'invention, et donc après formation des gouttes.

Selon un mode de réalisation, la solution d'augmentation de la viscosité comprend une base, notamment un hydroxyde d'alcalin, tel que l'hydroxyde de sodium.

Selon un mode de réalisation, lorsque le FI comprend au moins un corps gras solide à température et pression ambiante tel que décrit précédemment, le procédé de préparation d'une dispersion selon l'invention peut en outre comprendre une étape de chauffage du fluide FI, à une température comprise de 40°C à 150°C, de préférence de 50°C à 90°C, préalablement à l'étape susmentionnée de formation des gouttes, et donc avant mélange/mise en contact de ladite phase grasse avec la phase aqueuse continue.

Ce mode de réalisation, dans le cas d'un procédé « non-microfluidique » tel que susmentionné, peut nécessiter le maintien de cette étape de chauffage après mélange/mise en contact de ladite phase grasse avec la phase aqueuse continue lors de l'agitation jusqu'à l'obtention de l'émulsion recherchée.

Ce mode de réalisation, dans le cas où l'émulsion est préparée selon un procédé microfluidique, est avantageux, notamment, en ce qu'il permet de s'affranchir de la présence du fluide intermédiaire décrit ci-dessus.

Selon ce mode de réalisation, le procédé de préparation peut en outre comprendre, entre l'étape de chauffage et l'étape de formation des gouttes, une étape consistant à abaisser la température du fluide FI, le cas échéant jusqu'à température ambiante.

### *Utilisations*

De manière préférée, l'émulsion selon l'invention est directement utilisable, à l'issue des procédés de préparation précités, à titre de composition, notamment cosmétique. L'émulsion selon l'invention, lorsque préparée au moyen d'un procédé micro-fluidique tel que décrit ci-dessus, est également utilisable à titre de composition, notamment cosmétique, après séparation des gouttes et redispersion de celles-ci dans une seconde phase appropriée.

L'invention concerne également l'utilisation d'une l'émulsion selon l'invention pour la préparation d'une composition, notamment cosmétique.

La présente demande décrit également une composition, notamment cosmétique, comprenant au moins une dispersion selon l'invention, en association avec un milieu physiologiquement acceptable.

De préférence, une composition selon l'invention ne comprend pas de tensio-actif.

Les émulsions ou compositions selon l'invention peuvent notamment être utilisées dans le domaine cosmétique.
Elles peuvent comprendre, outre les ingrédients susmentionnés, au moins un milieu physiologiquement acceptable.
Par "milieu physiologiquement acceptable", on entend désigner un milieu convenant particulièrement à l'application d'une composition de l'invention sur les matières kératiniques, notamment la peau, les lèvres, les ongles, les cils ou les sourcils, et de préférence la peau.
Le milieu physiologiquement acceptable est généralement adapté à la nature du support sur lequel doit être appliquée la composition, ainsi qu'à l'aspect sous lequel la composition doit être conditionnée.
Selon un mode de réalisation, le milieu physiologiquement acceptable est figuré directement par la phase continue aqueuse telle que décrite ci-dessus.
Selon un mode de réalisation, les émulsions ou compositions cosmétiques sont utilisées pour le maquillage et/ou le soin de matières kératiniques, notamment de la peau.
Les compositions cosmétiques peuvent être des produits de soin, de protection solaire, de nettoyage (démaquillage), d'hygiène ou de maquillage de la peau.
Ces compositions sont donc destinées à être appliquées notamment sur la peau.
Ainsi, la présente invention concerne également l'utilisation cosmétique non thérapeutique d'une émulsion ou composition cosmétique susmentionnée, comme produit de maquillage, d'hygiène, de nettoyage et/ou de soin de matières kératiniques, notamment de la peau.
Selon un mode de réalisation, les émulsions ou compositions de l'invention sont sous la forme d'un fond de teint, d'un démaquillant, d'un soin du visage et/ou du corps et/ou du cheveu, d'un soin anti-âge, d'un protecteur solaire, d'un soin peau grasse, d'un soin whitening, d'un soin hydratant, d'une BB cream, crème teintée ou fond de teint, d'un nettoyant visage et/ou corps, d'un gel douche ou d'un shampoing.
Une composition de soin selon l'invention peut être en particulier une composition solaire, une crème de soin, un sérum ou un déodorant.
Les émulsions ou compositions selon l'invention peuvent être sous diverses formes, notamment sous forme de crème, de baume, de lotion, de sérum, de gel, de gel-crème ou encore de brume.
A l'application sur une matière kératinique, en particulier la peau, d'une émulsion ou d'une composition selon l'invention, les gouttes de l'émulsion se déstabilisent très rapidement, typiquement sous le cisaillement généré par les doigts sur la matière kératinique, sans opposer de résistance. Ce comportement à l'application présente l'avantage de contraster avec l'aspect visuel solide et granulaire des gouttes de l'émulsion.
La présente invention concerne également un procédé non thérapeutique de traitement cosmétique d'une matière kératinique, comprenant une étape d'application sur la matière kératinique d'au moins une émulsion ou d'au moins une couche d'une composition cosmétique telles que définies ci-dessus.
En particulier, la présente invention concerne un procédé non thérapeutique de traitement cosmétique de la peau, comprenant une étape d'application sur la peau d'au moins émulsion telle que définie ci-dessus.
Les émulsions ou compositions selon l'invention présentent avantageusement des viscosités compatibles avec une manipulation aisée du produit obtenu.
Dans toute la description, y compris les revendications, l'expression « comprenant un » doit être comprise comme étant synonyme de « comprenant au moins un », sauf si le contraire est spécifié.
Les expressions « compris entre ... et ... », « compris de ... à ... » et « allant de ... à ... » doivent se comprendre bornes incluses, sauf si le contraire est spécifié.
Les quantités des ingrédients figurant dans les exemples sont exprimées en pourcentage en poids par rapport au poids total de la composition, sauf indication contraire.
Les exemples qui suivent illustrent la présente invention sans en limiter la portée.

## EXEMPLES

Les polymères cationiques utilisés dans les exemples suivants sont les amodiméthicones suivantes :

| Amodiméthicone | Viscosité (en mPa.s) | Quantité de fonctions amines (en mmol par gramme d'amodiméthicone) |
|---|---|---|
| AM 1 (Nusil, CAS3131) | 1000 | 1,44 |

(suite)

| **Amodiméthicone** | **Viscosité** (en mPa.s) | **Quantité de fonctions amines** (en mmol par gramme d'amodiméthicone) |
|---|---|---|
| AM 2 (Nusil) | 3850 | 0,72 |
| AM 3 (Nusil) | 900 | 0,71 |

AM1 et AM3 présentent une viscosité similaire, mais une quantité de fonctions amines différentes, tandis que AM2 et AM3 présentent une quantité de fonctions amines similaire mais des viscosités différentes.

Protocole

Les émulsions des exemples 1 à 3 et 4 ci-après, sauf indication contraire, sont obtenues au moyen d'un protocole de préparation mettant en oeuvre un dispositif microfluidique, notamment tel que décrit dans WO/2012/120043 et WO/2015/055748.

**Exemple 1 : Préparation d'une dispersion de gouttes**

Les compositions des phases liquides permettant la préparation des dispersions (comparatives et selon l'invention) sont les suivantes :
La quantité d'amothiméthicone a été variée dans les dispersions préparées, conduisant ainsi à des émulsions selon l'invention (lorsque la quantité de fonctions amines, dans la phase grasse, est comprise entre 0,2 µmol et 10,5 µmol par gramme de phase grasse) et des dispersions comparatives (gammes en dehors de celle selon l'invention).

| | | | **Dispersion A** | **Dispersion B** | **Dispersion C** |
|---|---|---|---|---|---|
| **Fluide** | **Nom commercial** | **INCI** | % **poids** | % **poids** | % **poids** |
| OF | Eau osmosée | Aqua | 96,495 | 96,495 | 96,495 |
| | Microcare PTG | Pentylenglycol | 2,5 | 2,5 | 2,5 |
| | Microcare PE | Phenoxyethanol | 1 | 1 | 1 |
| | Carbomer Tego® 340FD | Carbomer | 0,25 | 0,25 | 0,25 |
| | Sodium hydroxyde pellets PRS codex | Sodium hydroxyde | 0,005 | 0,005 | 0,005 |
| | **Total** | | 100,000 | 100,000 | 100,000 |
| MF | Lanol 99 | Isononyl isononanoate | 100,000 | 100,000 | 100,000 |
| IF | Nusil CAS 3131 | Amodiméthicone AM1 | **0,026 à 0,859** | | |
| | | Amodiméthicone AM2 | | **0,052 à 1,70** | |
| | | Amodiméthicone AM3 | | | **0,0537 à 1,5** |
| | Lanol 99 | Isononyl isononanoate | qsp | qsp | qsp |
| | **Total** | | 100,000 | 100,000 | 100,000 |
| BF | Hydroxyde de sodium | Sodium hydroxide | 0,2 | 0,2 | 0,2 |
| | Eau osmosée | Aqua | qsp | qsp | qsp |
| Qsp : quantité suffisante pour | | | | | |

Les phases aqueuses (OF) obtenues possèdent une viscosité comprise entre 620 et 670mPa.s (10rpm) et un pH compris entre 4,60 et 4,75.

Les débits de phases utilisés dans le dispositif microfluidique sont les suivants :

| Phase | Débits (mL/hr) |
|---|---|
| OF | 100 |
| MF | 3,2825 |
| IF | 9,8475 |
| BF* | 10 |
| *Uniquement pour les tests de résistance mécanique. | |

La géométrie de la buse du dispositif microfluidique a été fixé avec pour diamètre interne de la tige inox, 1mm et le diamètre interne du canal d'OF, 1,8 mm.

**Exemple 2** : **Test de coalescence**

Des tests de coalescence ont été effectués sur les dispersions préparées dans l'exemple 1, comprenant les amodiméthicones AM1 (dispersions A), AM2 (dispersions B) ou AM3 (dispersions C).
La quantification de ce paramètre de coalescence a été effectuée par observation visuelle à l'aide d'un système de notation allant de 0 à 4. La note 0 se traduit par l'absence de coalescence et la note 4 décrit une très forte coalescence et la présence de gouttes ayant un diamètre supérieur à 2 mm.
L'échelle de notation utilisée est la suivante :

| Note | Critères de notation |
|---|---|
| 0 | Aucune coalescence |
| 1 | 1 à 3 gouttes coalescées |
| 2 | 4 à 10 gouttes coalescées |
| 3 | Plus de 10 gouttes coalescées |
| 4 | Présence de grosses gouttes (diamètre > 2mm) |

Pour représenter l'évolution de la coalescence, la moyenne des notes obtenues au cours des 3 mois de suivi a été représentée en fonction de la quantité de fonctions amines apportées par l'amodiméthicone dans l'IF des dispersions.
Cette quantité est exprimée en $\mu$mol d'amine par gramme de phase grasse selon la formule suivante :

$$Q = A \times T$$

avec :

- Q représente la quantité de fonctions amines apportées par le polymère cationique dans la phase grasse (en $\mu$mol/g),
- A représente le pourcentage massique du polymère cationique considéré dans la phase grasse, et
- T représente le taux de fonctions amines portées par ledit polymère cationique considéré (en $\mu$mol/g de polymère cationique), notamment obtenu par la méthode décrite ci-dessus).

Les résultats obtenus à température ambiante et 50°C sont présentés en figures 1 et 2 ci-après.
La figure 1 décrit l'évolution de la coalescence (moyenne des notes obtenues au cours des 3 mois de suivi) en fonction de la quantité de fonctions amines apportées par l'amodiméthicone (AM1, AM2 ou AM3) dans la phase grasse à température ambiante.
La figure 2 décrit l'évolution de la coalescence (moyenne des notes obtenues au cours des 3 mois de suivi) en fonction de la quantité de fonctions amines apportées par l'amodiméthicone (AM1, AM2 ou AM3) dans la phase grasse à 50°C.
Sur ces figures 1 et 2, les courbes des 3 amodiméthicones testés (AM1, AM2 et AM3) présentent la même évolution. Ces amodiméthicones ont donc un comportement similaire face à la coalescence. Ainsi, on peut en déduire

que les différences structurales des amodiméthicones ne semblent pas impacter la coalescence. En revanche, il a été observé que la coalescence est fonction de la quantité de fonctions amines (apportées par l'amodiméthicone) dans la phase grasse.

Des coalescences améliorées, c'est-à-dire faibles, voire nulles, ont notamment été observées pour les dispersions dont la quantité en fonctions amines (apportée par l'amodiméthicone) dans la phase grasse est comprise entre 0,2 μmol et 7 μmol par gramme de phase grasse (dispersions selon l'invention), et ce quelle que soit le nature de l'amodiméthicone. Ces résultats démontrent avantageusement la stabilité des émulsions selon l'invention.

Il a été démontré que la coalescence est d'autant plus faible, voire nulle, pour des émulsions dont la quantité en fonctions amines (apportée par l'amodiméthicone) dans la phase grasse est inférieure à 7 μmol, voire même inférieure à 5 μmol, et en particulier inférieure à 3 μmol, par gramme de phase grasse.

En particulier, des coalescences faibles, voire nulles, sont observées pour les dispersions dont la quantité en fonctions amines (apportée par l'amodiméthicone) dans la phase grasse est comprise entre 0,2 μmol et 5 μmol par gramme de phase grasse, de préférence entre 0,2 et 4. Les meilleurs résultats sont observés pour les dispersions dont la quantité en fonctions amines (apportée par l'amodiméthicone) dans la phase grasse est comprise entre 0,2 μmol et 3 μmol par gramme de phase grasse, et en particulier entre 0,2 et 2.

Au-dessus de 7 μmol, par gramme de phase grasse, les résultats montrent que la coalescence est induite de manière plus significative. Cette augmentation est d'ailleurs d'autant plus marquée pour les échantillons placés à 50°C. Ainsi, lorsque la quantité de fonctions amines apportées par l'amodiméthicone, dans la phase grasse, est supérieure à 7 μmol, les dispersions présentent une coalescence plus importante, et donc une stabilité moindre par rapport aux dispersions selon l'invention.

**Exemple 3 : Test de résistance mécanique**

Dans le cadre de cet exemple, la résistance mécanique des émulsions décrites en exemple 1 ci-dessus a été évaluée à l'aide du protocole suivant : un pot en verre de 4,5cm de diamètre et de 7 cm de hauteur contenant les dispersions à hauteur de 50mL, a été positionné sur un agitateur modulaire à rouleaux (R2P - Wheaton). En imposant une rotation du pot, les gouttes sont soumises à une combinaison de déformations et relaxations. Il s'agit à la fois d'un cisaillement simple mais également de phénomènes de compression et de dilation.

Les échantillons ont été soumis à un premier cycle de 3 minutes à 6 tpm puis a un deuxième cycle à 30 tpm de même durée. Après chaque cycle, une évaluation visuelle a été effectuée sur les échantillons avec le système de notation suivant : de 0, pour aucune fragmentation, à 4 pour une fragmentation des gouttes très importante. Ce test a été réalisé 1 mois après la production des gouttes.

Ce test de roulements a permis d'évaluer la résistance aux cisaillements des gouttes des émulsions.

Les résultats sont présentés en figures 3 et 4 ci-après.

La figure 3 décrit l'évaluation de la résistance mécanique (notation visuelle) en fonction de la quantité de fonctions amines apportées par l'amodiméthicone (AM1, AM2 ou AM3) dans la phase grasse (en μmol par gramme de phase grasse) à 6 tpm pendant 3 minutes.

La figure 4 décrit l'évaluation de la résistance mécanique (notation visuelle) en fonction de la quantité de fonctions amines apportées par l'amodiméthicone (AM1, AM2 ou AM3) dans la phase grasse (en μmol par gramme de phase grasse) à 30 tpm pendant 3 minutes.

Les résultats ont montré que, quelle que soit la nature de l'amodiméthicone étudiée, le comportement observé est similaire.

Il a été montré que pour des quantités en fonctions amines apportées par l'amodiméthicone, dans la phase grasse, trop faibles, typiquement inférieures à 0,2 μmol, la résistance mécanique des gouttes est très faible (une fragmentation importante est observée). Des fragmentations importantes des gouttes sont également observées pour des dispersions comparatives comprenant des quantités élevées en fonctions amines, par exemple supérieures à 10,5 μmol, dans la phase grasse.

Entre ces deux extrêmes, il a avantageusement été observé une zone où la résistance mécanique des gouttes est satisfaisante.

Malgré une bonne stabilité en condition statique, les dispersions obtenues en l'absence d'amine présentent une résistance mécanique nulle. Ainsi sous écoulement, la stabilité de cet échantillon n'est pas acceptable. Dans un contexte d'industrialisation, l'émulsion est soumise à des contraintes d'écoulement qui ne doivent pas provoquer la fragmentation des gouttes.

Or, il a été observé que la résistance mécanique est améliorée par l'augmentation de la quantité de fonctions amines (apportées par l'amodiméthicone) dans la phase grasse.

Ainsi, les émulsions selon l'invention comprenant une quantité en fonctions amines apportées par l'amodiméthicone, dans la phase grasse, comprise entre 0,2 μmol et 10,5 μmol par gramme de phase grasse, présentent avantageusement une résistance à la fragmentation des gouttes améliorée, ce qui traduit une résistance mécanique élevée

pour ces émulsions.

Cette résistance à la fragmentation des gouttes est encore améliorée pour des émulsions selon l'invention comprenant une quantité en fonctions amines apportées par l'amodiméthicone, dans la phase grasse, comprise entre 0,3 μmol et 7 μmol, de préférence entre 0,4 μmol et 5 μmol, et mieux entre 0,8 μmol et 2 μmol, par gramme de phase grasse

**Exemple 4** : **baume pailleté pour le corps**

Protocole de préparation (procédé non-microfluidiaue)

- Dans un bécher (1), peser l'eau osmosée et le(s) agent(s) chélatant(s) (notamment EDETA BD).

Placer le bécher (1) sous agitation mécanique avec une pale défloculeuse jusqu'à homogénéisation.

Arrêter l'agitation et ajouter dans le bécher (1) le(s) polymère(s) anionique(s) (notamment le carbomère).

Laisser le mélange obtenu au repos pendant environ 20 minutes (assure l'hydratation du polymère anionique), puis reprendre l'agitation jusqu'à homogénéisation.

Peser et ajouter dans le bécher (1) le(s) agent(s) gélifiant(s) de phase aqueuse (notamment Aristoflex AVC, Sepimax zen).

Le cas échéant, peser et ajouter une solution de soude 10%.

Agiter jusqu'à homogénéisation.

- Dans un autre bécher (2), peser la glycérine, le cas échéant avec au moins un agent gélifiant de phase aqueuse.

Mélanger à la spatule afin d'avoir un prémix homogène.

Ajouter le prémix (2) dans le bécher (1).

Quand la solution obtenue dans le bécher (1) est homogène, chauffer ladite solution à 67°C.

- Dans un autre bécher (3), peser le polymère cationique (notamment l'amodiméthicone) et l'isononanoate d'isononyle.

Sous agitation magnétique, chauffer la solution à 67°C pendant environ 5 minutes jusqu'à homogénéisation.

Puis, toujours dans le bécher (3), peser les éventuelles huiles et/ou corps gras solides additionnels.

Mettre le tout sous agitation magnétique, chauffer à 67°C pendant 10 minutes jusqu'à homogénéisation.

- Lorsque les solutions comprises dans les béchers (1) et (3) sont à température (i.e. 67°C), soumettre la solution du bécher (1) à une forte agitation mécanique.

L'obtention de l'émulsion consiste alors à ajouter sous cette forte agitation mécanique la solution du bécher (3) dans le bécher (1).

Laisser agiter pendant 15 à 20 minutes, puis refroidir à 40°C-35°C.

Dans un autre bécher (4), ajouter le(s) conservateur(s), notamment le phénoxyéthanol (i.e. Microcare PE) et/ou le pentylène glycol (i.e. Microcare Emollient PTG).

Mélanger à la spatule afin d'avoir un prémix homogène.

A 35°C, ajouter le prémix du bécher (4) dans le bécher (1).

- Lorsqu'il est présent, peser et ajouter l'alcool dénaturé (notamment l'alcool éthylique surfin 99 denat) dans le bécher (1).

Laisser homogénéiser.

- Peser et ajouter successivement dans le bécher (1) les actifs cosmétiques et/ou parfums et/ou agents colorants.

Laisser homogénéiser.

- Peser et ajouter une solution de soude 10% dans le bécher (1).

Un baume pour le corps est préparé selon le protocole décrit ci-dessus et comprend les ingrédients suivants :

| Dénomination commerciale | Fournisseur | INCI | % |
|---|---|---|---|
| Eau osmosée | Capsum | AQUA | 52,460% |
| EDETA BD | BASF | DISODIUM EDTA | 0,030% |
| Microcare PE | Thor | PHENOXYETHANOL, AQUA | 0,800% |
| Microcare Emollient PTG | Thor | PENTYLENE GLYCOL, AQUA | 2,000% |
| Tego Carbomer 340 FD | Evonik | CARBOMER | 0,192% |
| Carbopol Ultrez 21 polymer | Lubrizol | | 0,408% |
| Sepimax Zen | Seppic | POLYACRYLATE CROSSPOLYMER-6 | 0,250% |
| Glycérine codex (99%) | Interchimie | GLYCERIN | 20,000% |
| Zemea Propanediol | Dupont Tate & Lyle | PROPANEDIOL | 5,000% |
| Butylène glycol 1,3 | Interchimie | BUTYLENE GLYCOL | 5,000% |
| Solution soude 10% | Panreac | AQUA, SODIUM HYDROXYDE | 0,100% |
| Dub ININ | Nusil | ISONONYL ISONONANOATE | 2,000% |
| CAS-3131 | Nusil | AMODIMETHICONE | 0,020% |
| Dub PTB | Stéarinerie Dubois | PENTAERYTHRITYL TETRABEHENATE | 0,500% |
| Eutanol G | | | 1,000% |
| Lipex Shea Light | AAK | SHEA BUTTER ETHYL ESTERS | 1,000% |
| Plantée refined shea butter | CRM International | BUTYROSPERMUM PARKII | 0,500% |
| MOD | Gattefossé | | 1,500% |
| Lipocire A | Gattefossé | TRIGLYCERIDES C10-18 | 1,000% |
| Floraesters 30 | Floratech | JOJOBA ESTERS | 0,500% |
| Lanette O OR | BASF | CETEARYL ALCOHOL | 1,000% |
| Dub VCI 1 0 | Stéarinerie Dubois | ISODECYL NEOPENTANOATE | 1,000% |
| Sensual Drop | Givaudan | FRAGRANCE | 0,300% |
| Sepimat SB | Seppic | METHYL MATHACRYLATE CROSSPOLYMER | 2,000% |
| Solution soude 10% | Panreac | AQUA, SODIUM HYDROXYDE | 0,900% |
| Prestige soft bronze | Eckart | | 0,040% |
| Colorona Sun Gold Sparkle MP-29 | Merck | | 0,100% |
| Sunshine spectral gold | SunChemical | | 0,400% |

**Exemple 5** : **sérum anti-âge**

Le sérum anti-âge présentant les ingrédients suivants a été préparé.

| Nom | Nom INCI | % *w/w PHASES* | % w/w |
|---|---|---|---|
| PHASE GEL AQUEUX | | | |
| Eau osmosée | Aqua | 86,56 | 78,70 |
| Microcare PE | Phenoxyethanol | 0,88 | 0,80 |
| Microcare Emollient PTG | Pentylene glycol | 2,20 | 2,00 |
| Tego Carbomer 340FD | Carbomer | 0,27 | 0,25 |
| Rhodicare T | Xanthan gum | 0,11 | 0,10 |
| Phylcare Sodio Yaluronato XS | Sodium hyaluronate | 0,01 | 0,010 |
| Glycérine codex (99%) | Glycerin | 4,40 | 4,00 |
| Zemea | Propanediol | 5,50 | 5,00 |
| EDETA BD | Disodium EDTA | 0,03 | 0,030 |
| Solution 10% Sodium Hydroxide Pellets PRS codex | Aqua ; sodium hydroxide | 0,04 | 0,038 |
| Total | | *100,00* | *90,93* |
| PHASE GRASSE | | | |
| DUB ININ | Isononyl Isononanoate | 54,68 | 4,96 |
| KF-96A-50CS (PDMS 50cSt) | Dimethicone | 44,68 | 4,06 |
| Ionol CP | BHT (hydroxytoluène butylé) | 0,45 | 0,040 |
| **D&C Red N°17 K7007** | **CI 26100** | 0,0013 | 0,00012 |
| Nusil CAS 3131 | Amodimethicone | 0,18 | 0,020 |
| Total | | *100,00* | *9,08* |
| Total | | | 100,00 |

La composition finale comprend des gouttes de phase grasse rose translucides dispersées dans un gel aqueux incolore.

## Revendications

1. Emulsion comprenant une phase aqueuse continue et une phase grasse dispersée sous forme de gouttes, ou inversement, lesdites gouttes comprenant une écorce formée d'au moins un polymère anionique comprenant au moins une fonction acide carboxylique et d'au moins un polymère cationique comprenant au moins deux fonctions amines,

   dans laquelle la quantité de fonctions amines apportées par le polymère cationique, dans la phase grasse, est comprise entre 0,2 μmol et 2 μmol par gramme de phase grasse,
   le polymère cationique étant un polymère silicone modifié par une fonction amine primaire, secondaire ou tertiaire, et
   la phase grasse ne comprenant pas de polydiméthylsiloxane.

2. Emulsion selon la revendication 1, dans laquelle la phase grasse des gouttes ne comprend pas d'huile de silicone.

3. Emulsion selon la revendication 1 ou 2, dans laquelle l'émulsion est de type huile-dans-eau.

4. Emulsion selon l'une quelconque des revendications 1 à 3, dans laquelle le polymère cationique est l'amodiméthicone.

**5.** Emulsion selon l'une quelconque des revendications 1 à 4, dans laquelle le polymère cationique répond à la formule (I) suivante :

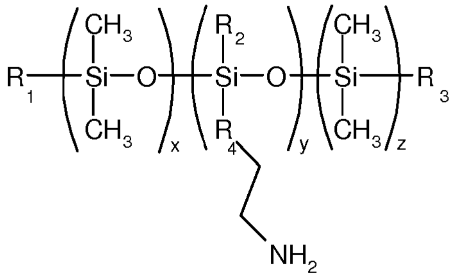

(I)

dans laquelle :

- $R_1$, $R_2$ et $R_3$, indépendamment les uns des autres, représentent OH ou $CH_3$ ;
- $R_4$ représente un groupe $-CH_2-$ ou un groupe -X-NH- dans lequel X est un radical alkylène divalent en C3 ou C4 ;
- x est un nombre entier compris entre 10 et 5 000, de préférence entre 30 et 1 000, et préférentiellement entre 80 et 300 ;
- y est un nombre entier compris entre 2 et 1 000, de préférence entre 4 et 100, et préférentiellement entre 5 et 20 ; et
- z est un nombre entier compris entre 0 et 10, de préférence entre 0 et 1.

**6.** Emulsion selon la revendication 5, dans laquelle le polymère cationique répond à la formule (I-1) suivante :

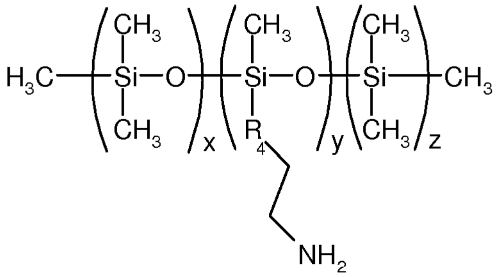

(I-1)

dans laquelle :

- $R_4$ est tel que défini dans la revendication 3, et est de préférence un groupe $-(CH_2)_3-NH-$ ;
- x est un nombre entier compris entre 80 et 300, de préférence entre 100 et 200 ;
- y est un nombre entier compris entre 5 et 20, de préférence entre 5 et 15 ; et
- z est un nombre entier compris entre 0 et 1, et de préférence égal à 1.

**7.** Emulsion selon l'une quelconque des revendications 1 à 6, dans laquelle la quantité de fonctions amines présentes dans le polymère cationique, et notamment dans l'amodiméthicone, est comprise entre 0,01 et 12,3, de préférence entre 0,1 et 8, plus préférentiellement entre 0,5 et 4, mmol, voire entre 0,5 et 2 mmol, par gramme dudit polymère cationique.

**8.** Emulsion selon l'une quelconque des revendications 1 à 7, dans laquelle la phase grasse comprend de 0,01% à 10%, de préférence de 0,05% à 5%, en poids de polymère(s) cationique(s) par rapport au poids total de la phase grasse.

**9.** Emulsion selon l'une quelconque des revendications 1 à 8, dans laquelle le polymère anionique est un carbomère ou un copolymère réticulé acrylates/$C_{10-30}$ alkyl acrylate, de préférence un carbomère.

**10.** Emulsion selon l'une quelconque des revendications 1 à 9, dans laquelle ladite émulsion comprend de 0,01% à 5%, de préférence de 0,05% à 2%, et préférentiellement de 0,10% à 0,5%, en poids de polymère(s) anionique(s), notamment de carbomère(s), par rapport au poids total de ladite émulsion.

**11.** Emulsion selon l'une quelconque des revendications 1 à 10, comprenant de 0,0001% à 50%, de préférence de 0,1% à 40%, et mieux de 1% à 25%, en poids d'huile(s) par rapport au poids total de ladite émulsion.

**12.** Emulsion selon l'une quelconque des revendications 1 à 11, comprenant en outre au moins un actif choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants et/ou les agents anti-âge, les agents parfumant, et leurs mélanges.

**13.** Utilisation d'une émulsion selon l'une quelconque des revendications 1 à 12, pour la préparation d'une composition cosmétique.

**14.** Procédé non thérapeutique de traitement cosmétique d'une matière kératinique, en particulier de la peau, comprenant au moins une étape d'application sur la matière kératinique d'au moins une émulsion selon l'une quelconque des revendications 1 à 12.

**Patentansprüche**

**1.** Emulsion, umfassend eine kontinuierliche wässrige Phase und eine dispergierte Fettphase in Form von Tropfen oder umgekehrt, die Tropfen umfassend eine Schale, die aus mindestens einem anionischen Polymer, umfassend mindestens eine Carbonsäurefunktion, und mindestens einem kationischen Polymer, umfassend mindestens zwei Aminfunktionen, gebildet ist,

wobei die Menge an Aminfunktionen, die durch das kationische Polymer in die Fettphase eingebracht werden, zwischen 0,2 µmol und 2 µmol pro Gramm Fettphase liegt,
wobei das kationische Polymer ein Silikonpolymer ist, das durch eine primäre, sekundäre oder tertiäre Aminfunktion modifiziert ist, und,
wobei die Fettphase kein Polydimethylsiloxan umfasst.

**2.** Emulsion nach Anspruch 1, wobei die Fettphase der Tropfen kein Silikonöl umfasst.

**3.** Vorrichtung nach einem der Ansprüche 1 oder 2, wobei die Emulsion vom Typ Öl-in-Wasser ist.

**4.** Emulsion nach einem der Ansprüche 1 bis 3, wobei das kationische Polymer Amodimethicon ist.

**5.** Emulsion nach einem der Ansprüche 1 bis 4, worin das kationische Polymer der folgenden Formel (I) entspricht:

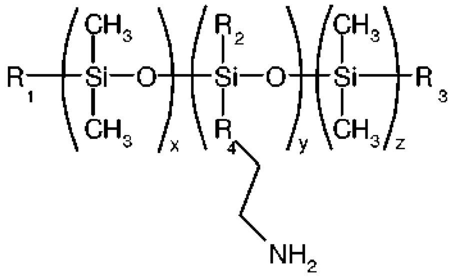

(I)

wobei:

- $R_1$, $R_2$ und $R_3$ unabhängig voneinander OH oder $CH_3$ darstellen;
- $R_4$ eine Gruppe $-CH_2-$ oder eine Gruppe -X-NH- darstellt, in der X ein zweiwertiger C3- oder C4-Alkylenradikal ist;
- x eine ganze Zahl zwischen 10 und 5000, vorzugsweise zwischen 30 und 1000 und besonders bevorzugt zwischen 80 und 300 ist;
- y eine ganze Zahl zwischen 2 und 1000, vorzugsweise zwischen 4 und 100 und besonders bevorzugt zwischen 5 und 20 ist; und
- z eine ganze Zahl zwischen 0 und 10, vorzugsweise zwischen 0 und 1, ist.

**6.** Emulsion nach Anspruch 5, wobei das kationische Polymer der folgenden Formel (1-1) entspricht:

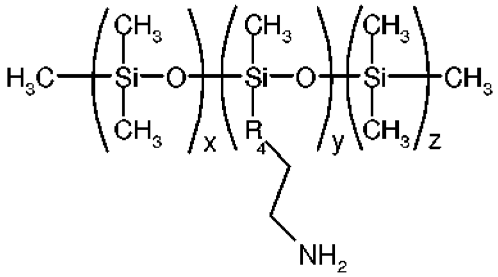

(I-1)

wobei:

- $R_4$ wie definiert in Anspruch 3 ist und vorzugsweise eine Gruppe $-(CH_2)_3$-NH- ist;
- x eine ganze Zahl zwischen 80 und 300, vorzugsweise zwischen 100 und 200, ist;
- y eine ganze Zahl zwischen 5 und 20, vorzugsweise zwischen 5 und 15, ist; und
- z eine ganze Zahl zwischen 0 und 1, vorzugsweise 1, ist.

**7.** Emulsion nach einem der Ansprüche 1 bis 6, wobei die Menge der in dem kationischen Polymer und insbesondere in dem Amodimethicon vorhandenen Aminfunktionen zwischen 0,01 und 12,3, vorzugsweise zwischen 0,1 und 8, bevorzugter zwischen 0,5 und 4 mmol und sogar zwischen 0,5 und 2 mmol pro Gramm des kationischen Polymers liegt.

**8.** Emulsion nach einem der Ansprüche 1 bis 7, wobei die Fettphase 0,01 bis 10 Gewichtsprozent, vorzugsweise 0,05 bis 5 Gewichtsprozent kationische(s) Polymer(e), bezogen auf das Gesamtgewicht der Fettphase, umfasst.

**9.** Emulsion nach einem der Ansprüche 1 bis 8, wobei das anionische Polymer ein Carbomer oder ein vernetztes Copolymer aus Acrylaten/C10-30-Alkylacrylat, vorzugsweise ein Carbomer, ist.

**10.** Emulsion nach einem der Ansprüche 1 bis 9, wobei die Emulsion 0,01 bis 5 Gewichtsprozent, vorzugsweise 0,05 bis 2 Gewichtsprozent und besonders bevorzugt 0,10 bis 0,5 Gewichtsprozent anionische(s) Polymer(e), insbesondere Carbomer(e), bezogen auf das Gesamtgewicht der Emulsion, umfasst.

**11.** Emulsion nach einem der Ansprüche 1 bis 10, umfassend von 0,0001 Gewichtsprozent bis 50 Gewichtsprozent, vorzugsweise 0,1 Gewichtsprozent bis 40 Gewichtsprozent und besonders bevorzugt 1 Gewichtsprozent bis 25 Gewichtsprozent Öl(e), bezogen auf das Gesamtgewicht der Emulsion.

**12.** Emulsion nach einem der Ansprüche 1 bis 11, ferner umfassend mindestens einen Wirkstoff, der ausgewählt ist aus feuchtigkeitsspendenden Mitteln, wundheilenden Mitteln, depigmentierenden Mitteln, UV-Filtern, schuppenlösenden Mitteln, Antioxidantien, die Synthese der dermalen und/oder epidermalen Makromoleküle stimulierenden Wirkstoffen, dermo-dekontrahierenden Mitteln, Antitranspiranten und/oder Anti-Aging-Mitteln, Duftstoffen und deren Gemischen.

**13.** Verwendung einer Emulsion nach einem der Ansprüche 1 bis 12 zur Herstellung einer kosmetischen Zusammensetzung.

**14.** Nicht therapeutisches Verfahren zur kosmetischen Behandlung eines Keratinmaterials, insbesondere der Haut, umfassend mindestens einen Schritt eines Auftragens mindestens einer Emulsion nach einem der Ansprüche 1 bis 12 auf das Keratinmaterial.

## Claims

**1.** Emulsion comprising a continuous aqueous phase and a dispersed fatty phase in the form of drops, or conversely, the drops comprising a shell formed of at least one anionic polymer comprising at least one carboxylic acid function and at least one cationic polymer comprising at least two amine functions,

wherein the quantity of amine functions provided by the cationic polymer in the fatty phase is between 0.2 μmol and 2 μmol per gram of fatty phase,
the cationic polymer being a silicone polymer modified with a primary, secondary or tertiary amine function, and wherein the fatty phase does not comprise polydimethylsiloxane.

2. Emulsion according to claim 1, wherein the fatty phase does not comprise silicone oil.

3. Emulsion according to claim 1 or 2, wherein the emulsion is of the oil-in-water type.

4. Emulsion according to any one of the claims 1 to 3, wherein the cationic polymer is amodimethicone.

5. Emulsion according to any one of the claims 1 to 4, wherein the cationic polymer has the following formula (I):

(I)
in which :

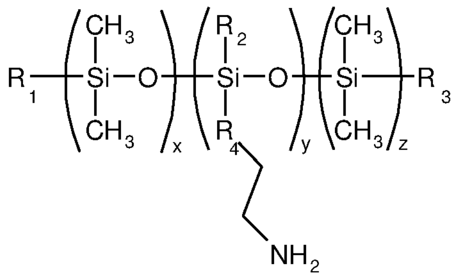

- $R_1$, $R_2$ and $R_3$, independently of each other, represent OH or $CH_3$;
- $R_4$ represents a group $-CH_2-$ or a group -X-NH- in which X is a divalent alkylene radical in $C_3$ or $C_4$;
- x is an integer between 10 and 5000, preferably between 30 and 1000, and more preferably between 80 and 300;
- y is an integer between 2 and 1000, preferably between 4 and 100, and more preferably between 5 and 20; and
- z is an integer between 0 and 10, preferably between 0 and 1.

6. Emulsion according to claim 5, wherein the cationic polymer has the following formula (I-1): (I-1 )
in which:

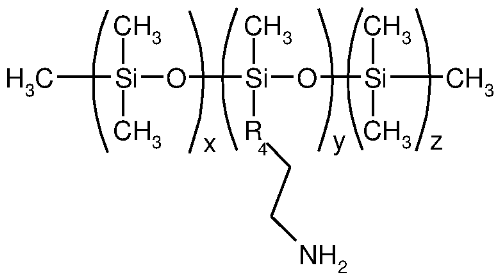

- $R_4$ is as defined in claim 3, and is preferably a $-(CH_2)_3-NH-$ group;
- x is an integer between 80 and 300, preferably between 100 and 200;
- y is an integer between 5 and 20, preferably between 5 and 15; and
- z is an integer between 0 and 1, and preferably equal to 1.

7. Emulsion according to any one of the claims 1 to 6, wherein the quantity of amine functions present in the cationic polymer, and in particular in the amodimethicone, is between 0.01 and 12.3, preferably between 0.1 and 8, more preferably between 0.5 and 4 mmol, or even between 0.5 and 2 mmol, per gram of the cationic polymer.

8. Emulsion according to any one of the claims 1 to 7, wherein the fatty phase comprises from 0.01% to 10%, preferably from 0.05% to 5%, by weight of cationic polymer(s) relative to the total weight of the fatty phase.

**9.** Emulsion according to any one of the claims 1 to 8, wherein the anionic polymer is a carbomer or a crosslinked copolymer acrylates/$C_{10-30}$ alkyl acrylate, preferably a carbomer.

**10.** Emulsion according to any one of the claims 1 to 9, wherein the emulsion comprises from 0.01% to 5%, preferably from 0.05% to 2%, and more preferably from 0.10% to 0.5%, by weight of anionic polymer(s), in particular of carbomer(s), relative to the total weight of the emulsion.

**11.** Emulsion according to any one of the claims 1 to 10, comprising from 0.0001% to 50%, preferably from 0.1% to 40%, and more preferably from 1% to 25%, by weight of oil(s) relative to the total weight of the emulsion.

**12.** Emulsion according to any one of the claims 1 to 11, further comprising at least one active agent selected from hydrating agents, healing agents, depigmenting agents, UV filters, desquamating agents, antioxidants, stimulating active agents for the synthesis of dermal and/or epidermal macromolecular agents, dermodecontracting agents, anti-perspirant agents and/or anti-aging agents, perfuming agents, and mixtures thereof.

**13.** Use of an emulsion according to any one of the claims 1 to 12 for the preparation of a cosmetic composition.

**14.** Non-therapeutic method for the cosmetic treatment of a keratin material, in particular the skin, comprising at least one step of applying to the keratin material at least one emulsion according to any one of the claims 1 to 12.

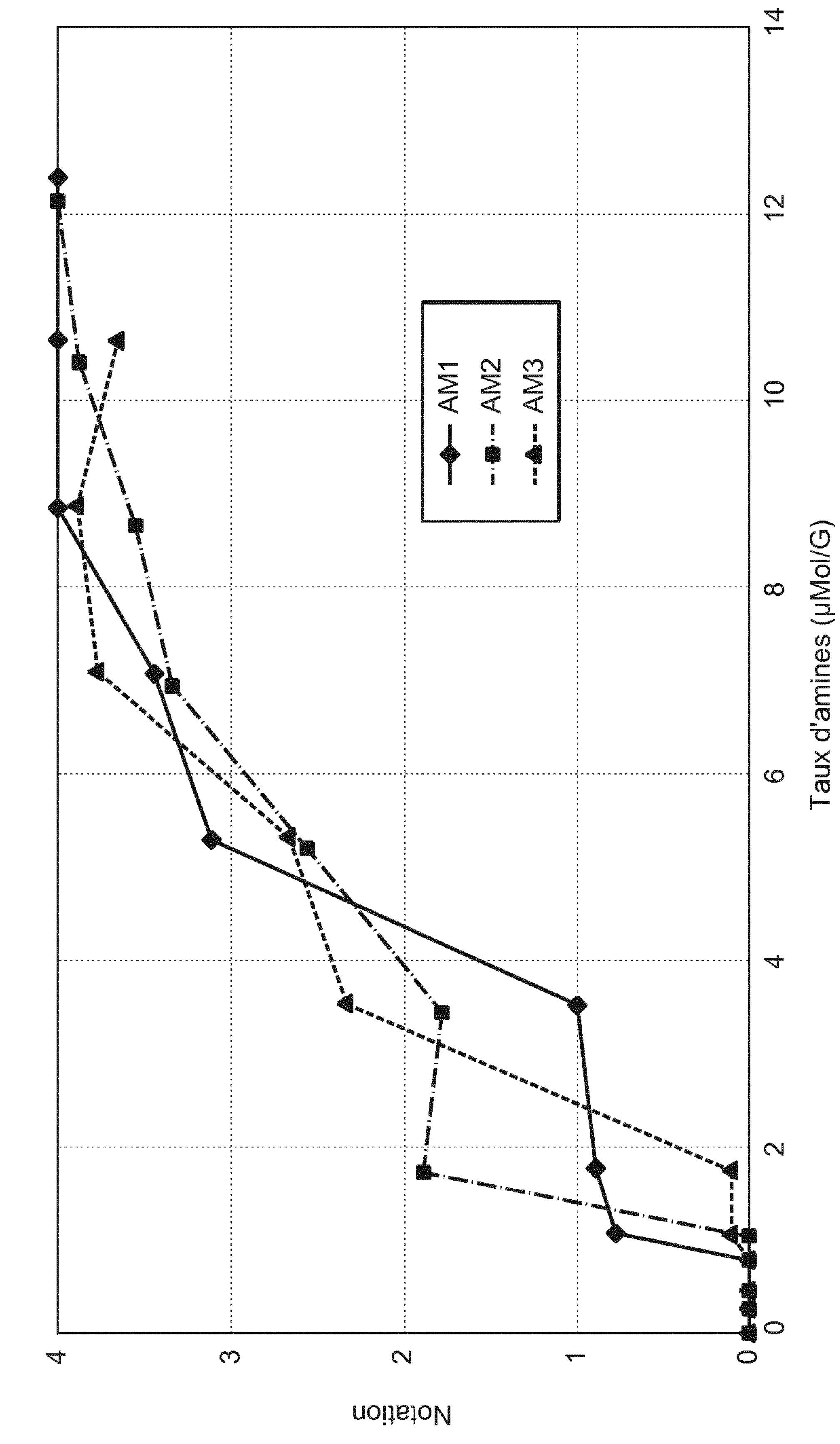


FIG.1

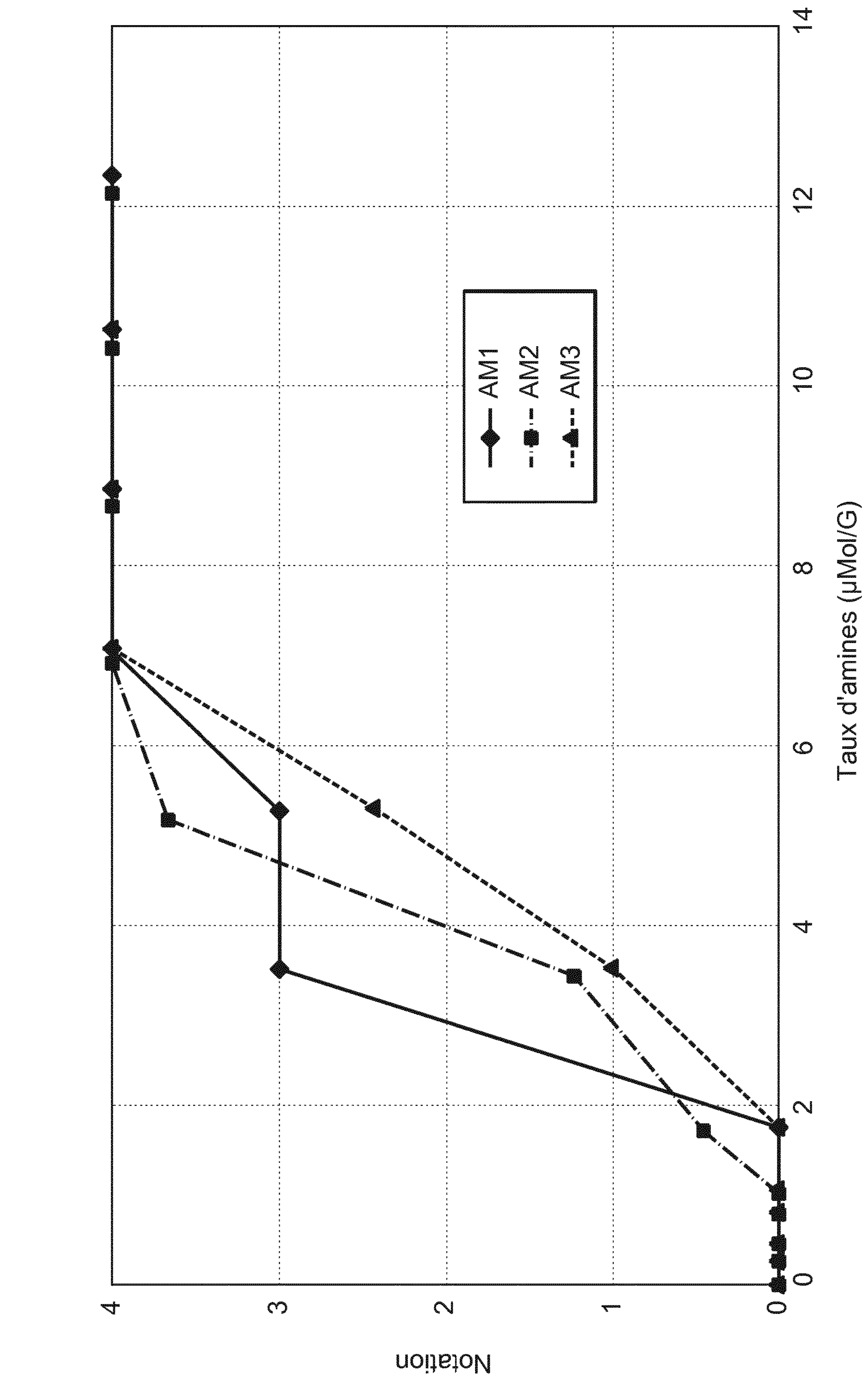


FIG.2

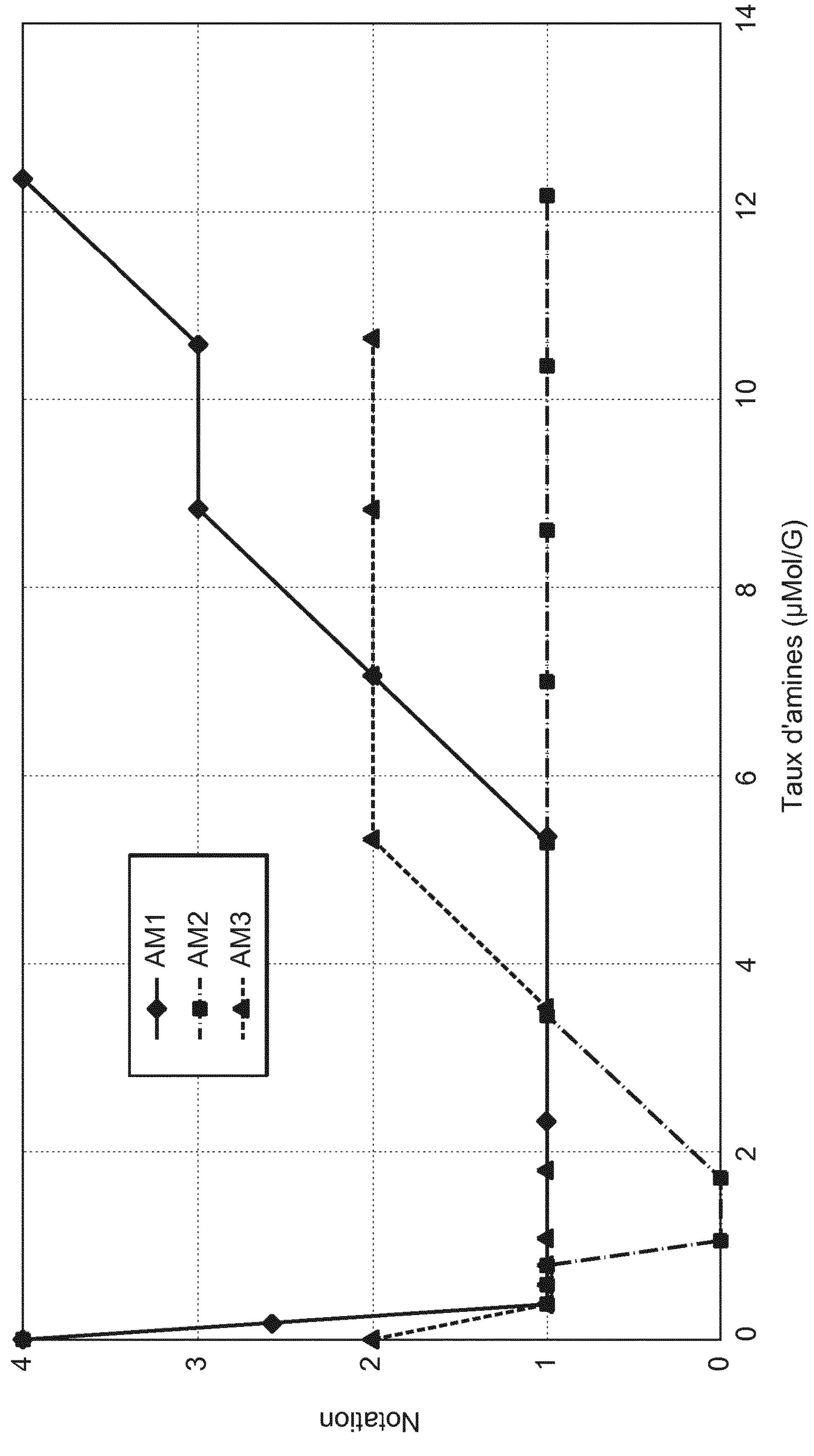


FIG.3

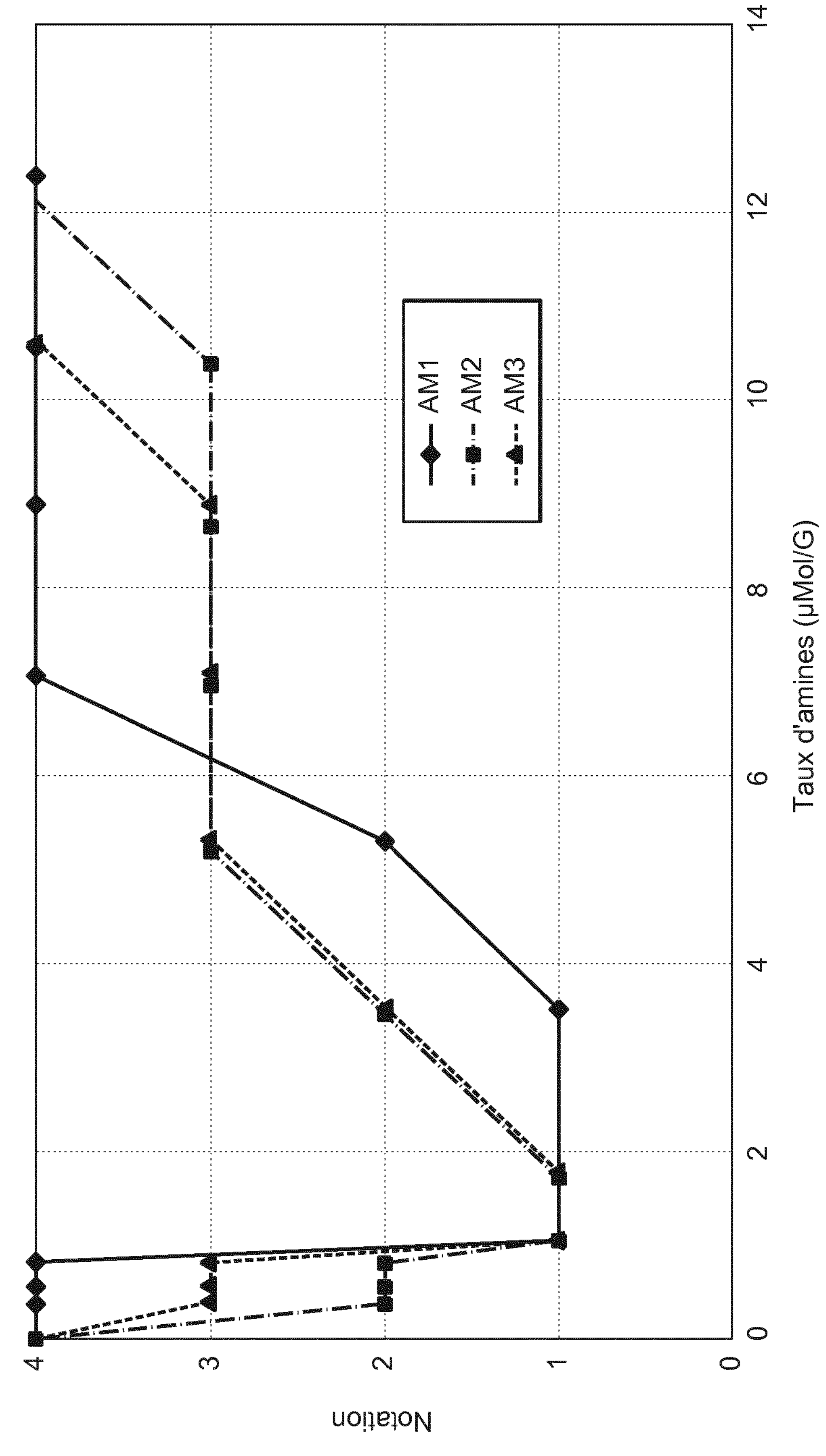


FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2012120043 A
- US 20140045949 A
- WO 2010063937 A
- JP 2295912 A
- FR 2792190 A
- WO 2015055748 A


### Littérature non-brevet citée dans la description


- Fats and Fatty Oils. **A. THOMAS.** Ullmann's Encyclopedia of Industrial Chemistry. 15 Juin 2000
- **SATO et al.** *J. Chem. Phys.,* 2007, vol. 111, 1393-1401
- handbook of Pharmaceutical Excipients